# EUROPEAN PATENT SPECIFICATION

(11) **EP 1 419 158 B1**
(45) Date of publication and mention of the grant of the patent: **01.08.2007**
(21) Application number: 02749129.9
(22) Date of filing: 09.08.2002
(51) Int. Cl.: C07D 475/06, A61K 31/505, A61P 19/02

(54) **PTERIDINONE DERIVATIVES AS MODULATORS OF CHEMOKINE RECEPTOR ACTIVITY**
PTERIDINON-DERIVATE ALS MODULATOREN DER CHEMOKINREZEPTOR-AKTIVITÄT
DERIVES DE PTERIDINONE SERVANT DE MODULATEURS DE L'ACTIVITE DE RECEPTEUR DE CHIMIOKINES

(30) Priority: 14.08.2001 SE 0102716
(43) Date of publication of application: 19.05.2004
(73) Proprietor: AstraZeneca AB, 151 85 (SE)
(72) Inventor: BONNERT, Roger, Victor, Loughborough, Leicestershire LE11 5RH (GB); CAGE, Peter, Alan, Loughborough, Leicestershire LE11 5RH (GB); HUNT, Simon, Frazer, Loughborough, Leicestershire LE11 5RH (GB); WALTERS, Iain, Alastair, Stewart, Loughborough, Leicestershire LE11 5RH (GB); AUSTIN, Rupert, Philip AstraZeneca R&D Chamwood, Loughborough, Leicester LE11 5RH (GB)
(86) International application number: PCT/GB2002/003684
(87) International publication number: WO 2003/024966

(56) References cited:
- WO-A-00/09511
- WO-A-01/19825
- WO-A-01/25242
- WO-A-01/58906
- WO-A-01/62758
- WO-A-02/32507

## Description

The present invention relates to certain heterocyclic compounds, processes and intermediates used in their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. At the present time, the chemokine superfamily comprises three groups exhibiting characteristic structural motifs, the Cys-X-Cys (C-X-C), Cys-Cys (C-C)) and Cys-X₃-Cys (C X₃-C) families. The C-X-C and C-C families have sequence similarity and are distinguished from one another on the basis of a single amino acid insertion between the NH-proximal pair of cysteine residues. The C-X₃-C family is distinguished from the other two families on the basis of having a triple amino acid insertion between the NH-proximal pair of cysteine residues.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils. Examples include human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

The C-X₃-C chemokine (also known as fractalkine) is a potent chemoattractant and activator of microglia in the central nervous system (CNS) as well as of monocytes, T cells, NK cells and mast cells.

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family. These receptors represent good targets for drug development since agents which modulate these receptors would be useful in the treatment of disorders and diseases such as those mentioned above.

WO-01/19825 discloses pteridinones as kinase inhibitors. WO-02/32507 discloses 7-amino-2-alkylthiopteridin-4-yl-amines for the treatment of chemokine related disorders. WO-01/62758 discloses pteridine compounds for the treatment of psoriasis. WO-01/58906 discloses pyrimidine compounds as modulators of chemokine receptor activity. WO-00/09511 and WO-01/25242 disclose thiazolopyrimidines.

The present invention therefore provides compounds of formula (I) and pharmaceutically acceptable salts, solvates or *in vivo* hydrolysable esters thereof: 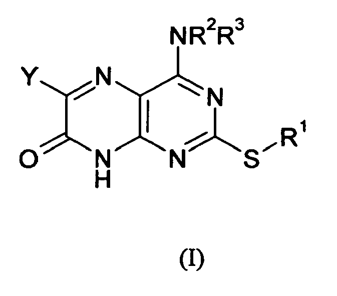 in which:
R¹ represents a C₃-C₇carbocyclic, C₁-C₈ alkyl, C₂-C₆ alkenyl or C₂-C₆ alkynyl group, each of which may be optionally substituted by one or more substituent groups independently selected from halogen atoms, -OR⁴, -NR⁵R⁶, -CONR⁵R⁶, -COOR⁷, -NR⁸COR⁹, -SR¹⁰, -SO₂R¹⁰, -SO₂NR⁵R⁶, -NR⁸SO₂R⁹, an aryl or heteroaryl group, which last two may themselves be optionally substituted by one or more substituents independently selected from halogen atoms, cyano, nitro, -OR⁴, -NR⁵R⁶, -CONR⁵R⁶, -COOR⁷, -NR⁸COR⁹, -SR¹⁰, -SO₂R¹⁰, -SO₂NR⁵R⁶ , -NR⁸SO₂R⁹, C₁-C₆ alkyl or trifluoromethyl groups;
R² and R³ each independently represent a hydrogen atom, or a C₃-C₇ carbocyclic, C₁-C₈ alkyl, C₂-C₆ alkenyl or C₂-C₆ alkynyl group, the latter four groups may be optionally substituted by one or more substituent groups independently selected from:
   (a) halogen atoms, -OR⁴, -NR⁵R⁶ -CONR⁵R⁶, - COOR⁷, - NR⁸COR⁹, - SR¹⁰, - SO₂R¹⁰, -SO₂NR⁵R⁶, -NR⁸SO₂R⁹;
   (b) a 3-8 membered ring optionally containing one or more atoms selected from O, S, NR⁸ and itself optionally substituted by C₁-C₃ alkyl or halogen; or
   (c) an aryl group or heteroaryl group each of which may be optionally substituted by one or more substituents independently selected from halogen atoms, cyano, nitro, -OR⁴, -NR⁵R⁶, - CONR⁵R⁶, -NR⁸COR⁹, -SO₂NR⁵R⁶, -NR⁸SO₂R⁹, C₁-C₆ alkyl and trifluoromethyl groups;
R⁴ represents hydrogen or a C₁-C₆ alkyl group which may be optionally substituted by one or more substituent groups independently selected from halogen atoms, -OR¹¹, -NR⁵R⁶, or an aryl group or heteroaryl group either of which may be optionally substituted by one or more substituents independently selected from halogen atoms, cyano, nitro, -OR¹¹, -NR⁵R⁶, - CONR⁵R⁶, -NR⁸COR⁹, -SO₂NR⁵R⁶, -NR⁸SO₂R⁹, C₁-C₆ alkyl and trifluoromethyl groups; or R⁴ represents a halogen atom, -OR¹¹, -NR⁵R⁶, or an aryl group or heteroaryl group either of which may be optionally substituted by one or more substituents independently selected from halogen atoms, cyano, nitro, -OR¹¹, -NR⁵R⁶, -CONR⁵R⁶, -NR⁸COR⁹, -SO₂NR⁵R⁶,-NR⁸SO₂R⁹, C₁-C₆ alkyl and trifluoromethyl groups;
R⁵ and R⁶ independently represent a hydrogen atom or a C₁-C₆ alkyl or phenyl group or heteroaryl group the latter three of which may be optionally substituted by one or more substituent groups independently selected from halogen atoms, phenyl, -OR¹⁴ and - NR¹⁵R¹⁶, -CONR¹⁵R¹⁶, -NR¹⁵COR¹⁶, -SONR¹⁵R¹⁶, NR¹⁵SO₂R¹⁶
   or
R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from phenyl, -OR¹⁴, -COOR¹⁴, -NR¹⁵R¹⁶, -CONR¹⁵R¹⁶, -NR¹⁵COR¹⁶, -SONR¹⁵R¹⁶, NR¹⁵SO²R¹⁶ or C₁-C₆ alkyl, itself optionally substituted by one or more substituents independently selected from halogen atoms and - NR¹⁵R¹⁶ and -OR¹⁷ groups;
R¹⁰ represents a C₁-C₆-alkyl or a phenyl group, either of which may be optionally substituted by one or more substituent groups independently selected from halogen atoms, phenyl, -OR¹⁷ and -NR¹⁵R¹⁶,
Y represents NR²⁰R²¹, OR⁴, SR⁴, a heteroaryl group or NR⁵R⁶ where R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from phenyl, -OR¹⁴, -COOR¹⁴, -NR¹⁵R¹⁶, -CONR¹⁵R¹⁶, -NR¹⁵COR¹⁶, -SONR¹⁵R¹⁶, NR¹⁵SO₂R¹⁶ or C₁-C₆ alkyl, itself optionally substituted by one or more substituents independently selected from halogen atoms and -NR¹⁵R¹⁶ and -OR¹⁷ groups;
each of R⁷, R⁸, R⁹, R¹¹, R¹⁴ R¹⁵, R¹⁶ and R¹⁷ independently represents a hydrogen atom or a C₁-C₆, alkyl, or a phenyl group;
and R²⁰ and R²¹ are defined as for R² and R³

The present invention further provides compounds of formula (I) and pharmaceutically acceptable salts or solvates thereof: 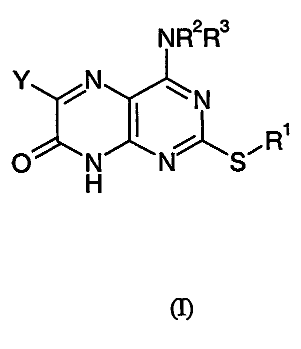 in which:
R¹ represents a C₃-C₇ carbocyclic, C₁-C₈ alkyl, C₂-C₆ alkenyl or C₂-C₆ alkynyl group, each of which may be optionally substituted by one or more substituent groups independently selected from halogen atoms, -OR⁴, -NR⁵R⁶, - CONR⁵R⁶, -COOR⁷, -NR⁸COR⁹, - SR¹⁰, - SO₂R¹⁰, - SO₂NR⁵R⁶, -NR⁸SO₂R⁹, an aryl or heteroaryl group, which last two may themselves be optionally substituted by one or more substituents independently selected from halogen atoms, cyano, nitro, -OR⁴, -NR⁵R⁶, -CONR⁵R⁶, -COOR⁷, -NR⁸COR⁹, -SR¹⁰, -SO₂R¹⁰, -SO₂NR⁵R⁶, -NR⁸SO₂R⁹, C₁-C₆ alkyl or trifluoromethyl groups;
R² and R³ each independently represent a hydrogen atom, or a C₃-C₇ carbocyclic, C₁-C₈ alkyl, C₂-C₆ alkenyl or C₂-C₆ alkynyl group, the latter four groups may be optionally substituted by one or more substituent groups independently selected from:
   (a) halogen atoms, -OR⁴, -NR⁵R⁶-CONR⁵R⁶, - COOR⁷, -NR⁸COR⁹, - SR¹⁰, -SO₂R¹⁰, -SO₂NR⁵R⁶ -NR⁸SO₂R⁹;
   (b) a 3-8 membered ring optionally containing one or more atoms selected from O, S, NR⁸ and itself optionally substituted by C₁-C₃-alkyl or halogen; or
   (c) an aryl group or heteroaryl group each of which may be optionally substituted by one or more substituents independently selected from halogen atoms, cyano, nitro, -OR⁴, -NR⁵R⁶, - CONR⁵R⁶, -NR⁸COR⁹, -SO₂NR⁵R⁶, -NR⁸SO₂R⁹, C₁-C₆ alkyl and trifluoromethyl groups;
R⁴ represents hydrogen or a C₁-C₆ alkyl group the latter of which may be optionally substituted by one or more substituent groups independently selected from halogen atoms, - OR¹¹, -NR⁵R⁶, or an aryl group or heteroaryl group either of which may be optionally substituted by one or more substituents independently selected from halogen atoms, cyano, nitro, -OR¹¹, -NR⁵R⁶, -CONR⁵R⁶, -NR⁸COR⁹, -SO₂NR⁵R⁶, -NR⁸SO₂R⁹, C₁-C₆ alkyl and trifluoromethyl groups;
R⁵ and R⁶ independently represent a hydrogen atom or a C₁-C₆ alkyl or phenyl group the latter two of which may be optionally substituted by one or more substituent groups independently selected from halogen atoms, phenyl, -OR¹⁴ and -NR¹⁵R¹⁶, -CONR¹⁵R¹⁶, -NR¹⁵COR¹⁶, - SONR¹⁵R¹⁶, NR¹⁵SO₂R¹⁶
   or
R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from phenyl, -OR¹⁴, -COOR¹⁴, -NR¹⁵R¹⁶, -CONR¹⁵R¹⁶, -NR¹⁵COR¹⁶, -SONR¹⁵R¹⁶, NR¹⁵SO₂R¹⁶ or C₁-C₆ alkyl, itself optionally substituted by one or more substituents independently selected from halogen atoms and - NR¹⁵R¹⁶ and -OR¹⁷ groups;
R¹⁰ represents a C₁-C₆-alkyl or a phenyl group, either of which may be optionally substituted by one or more substituent groups independently selected from halogen atoms, phenyl, -OR¹⁷ and -NR¹⁵R¹⁶,
Y is NR²⁰R²¹, OR⁴ or SR⁴;
each of R⁷, R⁸, R⁹, R¹¹, R¹², R¹³, R¹⁴ R¹⁵, R¹⁶, R¹⁷, R¹⁸ and R¹⁹ independently represents a hydrogen atom or a C₁-C₆, alkyl, or a phenyl group;
and R²⁰ and R²¹ are defined as for R² and R³.

In the context of the present specification, unless otherwise indicated, the term alkyl includes both straight-chain and branched-chain alkyl groups. However references to individual alkyl groups such as "propyl" are specific for the straight chain version only and references to individual branched-chain alkyl groups such as t-butyl are specific for the branched chain version only. Examples of C₁₋C₃ alkyl include methyl, ethyl, propyl. Examples of C₁₋C₆ alkyl include the examples of C₁-C₃ alkyl and additionally butyl, *t*-butyl, pentyl, 2-methylbutyl and hexyl. Examples of C₁-C₈ alkyl include the examples of C₁-C₆alkyl and additionally heptyl, 2-ethyl-3-methylbutyl and octyl. An analogous convention applies to other terms such as alkenyl and alkynyl. For example C₂-C₆ alkenyl includes vinyl, allyl, 1-propenyl, 2-butenyl, 2-methylbut-2-enyl, and 4-hexenyl. Examples of C₂-C₆ alkynyl include ethynyl, 1-propynyl, 2-propynyl and 1-methylpent-2-ynyl.

C₃-C₇ carbocyclic is a saturated, partially saturated or unsaturated ring system containing 3 to 7 ring carbon atoms. C₃-C₇ carbocyclic groups include cyclobutyl, cyclopentyl, cyclopentenyl, cyclohexyl and cyclohexenyl.

Examples of 4- to 7-membered saturated heterocyclic ring systems optionally containing a further heteroatom selected from oxygen and nitrogen atoms include azetidinyl, pyrrolidinyl, piperidinyl, morpholinyl and piperazinyl.

Aryl groups include phenyl and naphthyl. Heteroaryl is defined as a 5- or 6-membered aromatic ring containing one or more heteroatoms selected from N, S, O. Examples include pyridine, pyrimidine, thiazole, oxazole, pyrazole, imidazole, furan.
Further examples include pyridine, pyrimidine, thiazole, oxazole, pyrazole, imidazole, furan, triazole and thiadiazole.

Halogen atoms include fluorine, chlorine, bromine and iodine. Preferred halogen atoms are fluorine and chlorine.

Where a group is substituted or optionally substituted by one or more substitutents it is to be understood that this definition includes all substituents being chosen from one of the specified groups or the substituents being chosen from two or more of the specified groups. Preferably one or more means 1, 2 or 3. One or more may also mean 1 or 2. Where a ring contains or optionally contains one or more atoms, preferably it contains 1, 2, 3 or 4 atoms.

Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention.

The invention further encompasses all solvated forms of compounds of formula (I) and salts thereof.

Preferred values of R¹, R², R³ and Y are as follows. Such values may be used where appropriate with any of the definitions, claims or embodiments defined hereinbefore or hereinafter.

Suitably the group R¹ represents a C₃-C₇ carbocyclic, C₁-C₈ alkyl, C₂-C₆ alkenyl or C₂-C₆ alkynyl group, each of which may be optionally substituted by one or more substituent groups independently selected from halogen atoms, -OR⁴, -NR⁵R⁶, -CONR⁵R⁶, -COOR⁷, - NR⁸COR⁹, -SR¹⁰, -SO₂R¹⁰, -SO₂NR⁵R⁶, -NR⁸SO₂R⁹, an aryl or heteroaryl group both of which can be optionally substituted by one or more substituents independently selected from halogen atoms, cyano, nitro, -OR⁴, -NR⁵R⁶, -CONR⁵R⁶, -COOR⁷, -NR⁸COR¹⁰, -SR¹⁰, -SO₂R¹⁰, -SO₂NR⁵R⁶, -NR⁸SO₂R¹⁰, C₁-C₆ alkyl or trifluoromethyl groups. Particularly advantageous compounds of formula (I) are those in which R¹ represents an optionally substituted benzyl group. More preferably R¹ represents benzyl or benzyl substituted by one or more C₁-C₆ alkyl, C₁-C₆ alkoxy, or halogen atoms, in particular benzyl substituted by two halogen atoms.

Preferably one of R² and R³ is hydrogen and the other is C₁-C₈ alkyl substituted by hydroxy and one or more methyl or ethyl groups. More preferably one of R² and R³ is hydrogen and the other is CH(CH₃)CH₂OH, CH(Et)CH₂OH, C(CH₃)₂CH₂OH or CH(CH₂OH)₂. When one of R² and R³ is hydrogen and the other is CH(CH₃)CH₂OH or CH(Et)CH₂OH the resulting compounds of formula (I) are preferably in the form of the (R) isomer. Most preferably one of R² and R³ is hydrogen and the other is CH(CH₃)CH₂OH.

Preferably Y represents -NR²⁰R²¹, -OR⁴, -SR⁴, a heteroaryl group or -NR⁵R⁶ where R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from -OH, -NH₂ or C₁-C₄ alkyl.

Preferably one of R²⁰ and R²¹ is hydrogen or methyl and the other is a C₃-C₇carbocyclic substituted by hydroxy or it is C₁-C₄alkyl substituted by -OR⁴, heteroaryl optionally substituted by methyl, or a 3-8 membered ring optionally containing one or more atoms selected from O, S and NR⁸.

Preferably R⁴ represents hydrogen or a C₁-C₆ alkyl group the latter of which may be optionally substituted by -NR⁵R⁶ or an heteroaryl group which may be optionally substituted by one or more substituents independently selected from halogen atoms, cyano, nitro, -OR¹¹, -NR⁵R⁶, - CONR⁵R⁶, -NR⁸COR⁹, -SO₂NR⁵R⁶, -NR⁸SO₂R⁹, C₁-C₆ alkyl and trifluoromethyl groups; or R⁴ represents a heteroaryl group which may be optionally substituted by one or more substituents independently selected from -OH and methyl.

Preferably one of R⁵ and R⁶ is hydrogen and the other is C₁-C₆alkyl or a heteroaryl group; or together with the nitrogen atom to which they are attached R⁵ and R⁶ form a 4- to 7-membered saturated heterocyclic ring system optionally containing a further heteroatom selected from oxygen and nitrogen atoms, which ring system may be optionally substituted by one or more substituent groups independently selected from -OH, -NH₂ or C₁-C₄ alkyl.

A preferred class of compound is of formula (I) in which;
R¹ represents benzyl or benzyl substituted by one or more C₁-C₆ alkyl, C₁-C₆ alkoxy, or halogen atoms;
R² represents hydrogen;
R³ represents C₁-C₈ alkyl substituted by hydroxy and one or more methyl or ethyl groups;
Y represents -NR²⁰R²¹, -OR⁴, -SR⁴, a heteroaryl group or -NR⁵R⁶ ;
R²⁰ represents hydrogen or methyl;
R²¹ represents a C₃-C₇carbocyclic substituted by hydroxy; or C₁-C₄alkyl substituted by -OR⁴, heteroaryl (optionally substituted by methyl), or a 3-8 membered ring optionally containing one or more atoms selected from O, S and NR⁸;
R⁴ represents hydrogen or a C₁-C₆ alkyl group optionally substituted by -NR⁵R⁶ or an heteroaryl group optionally substituted by one or more substituents independently selected from halogen atoms, cyano, nitro, -OR¹¹, -NR⁵R⁶, -CONR⁵R⁶, -NR⁸COR⁹, -SO₂NR⁵R⁶, - NR⁸SO₂R⁹, C₁-C₆ alkyl and trifluoromethyl groups; or R⁴ represents a heteroaryl group optionally substituted by one or more substituents independently selected from -OH and methyl;
R⁵ represents hydrogen;
R⁶ represents C₁-C₆alkyl or a heteroaryl group;
or R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from -OH, -NH₂ or C₁-C₄ alkyl;
R⁸ represents a hydrogen atom or a C₁-C₆ alkyl or a phenyl group;
R⁹ represents a hydrogen atom or a C₁-C₆ alkyl or a phenyl group; and
R¹¹ represents a hydrogen atom or a C₁-C₆ alkyl or a phenyl group.

Another preferred class of compound is of formula (I) in which;
R¹ represents benzyl substituted by two halogen atoms;
R² represents hydrogen;
R³ represents CH(CH₃)CH₂OH, CH(Et)CH₂OH C(CH₃)₂CH₂OH or CH(CH₂OH)₂;
Y represents - NR²⁰R²¹, - OR⁴, - SR⁴, a heteroaryl group or -NR⁵R⁶;
R²⁰ represents hydrogen or methyl;
R²¹ represents a C₃-C₇carbocyclic substituted by hydroxy; or C₁-C₄alkyl substituted by -OR⁴, heteroaryl (optionally substituted by methyl), or a 3-8 membered ring optionally containing one or more atoms selected.from O, S and NR⁸;
R⁴ represents hydrogen or a C₁-C₆ alkyl group optionally substituted by -NR⁵R⁶ or an heteroaryl group optionally substituted by one or more substituents independently selected from halogen atoms, cyano, nitro, -OR¹¹, -NR⁵R⁶, -CONR⁵R⁶, -NR⁸COR⁹, -SO₂NR⁵R⁶, - NR⁸SO₂R⁹, C₁-C₆ alkyl and trifluoromethyl groups; or R⁴ represents a heteroaryl group optionally substituted by one or more substituents independently selected from -OH and methyl;
R⁵ represents hydrogen;
R⁶ represents C₁-C₆alkyl or a heteroaryl group;
   or R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from -OH, -NH₂ or C₁-C₄ alkyl;
R⁸ represents a hydrogen atom or a C₁-C₆ alkyl or a phenyl group;
R⁹ represents a hydrogen atom or a C₁-C₆ alkyl or a phenyl group; and
R¹¹ represents a hydrogen atom or a C₁-C₆ alkyl or a phenyl group.

Another preferred class of compound is of formula (I) in which;
R¹ represents benzyl substituted by two fluorine atoms;
R² represents hydrogen;
R³ represents CH(CH₃)CH₂OH;
Y represents (2-hydroxyethyl)amino, (phenylmethyl)amino, amino, 1*H*-imidazolyl, (1-methyl-1*H*-imidazolyl)thio, methoxy, (3-pyridylmethyl)amino, [(5-methyl-2-furanyl)methyl]amino, 3,5-dimethyl-1-piperazinyl, N-methyl-N-[(3-methyl-5-isoxazolyl)methyl]amino, [2-(2-pyrimidinylamino)ethyl]amino, 4-morpholinyl, [2-(4-morpholinyl)ethyl]amino, (2-methoxyethyl)amino, (2-furanylmethyl)amino, 1-azetidinyl, [(5-methylpyrazinyl)methyl]amino, [2-(2-furanyl)ethyl]amino, [3-(4-morpholinyl)propyl]amino, [3-methyl-5-isoxazolyl)methyl]amino, 3-hydroxy-1-pyrrolidinyl, (2-furanylmethyl)thio, (2-hydroxypropyl)amino, [2-(dimethylamino)ethyl]thio, (2-hydroxypropyl)amino, (3-hydroxypropyl)amino, N-(2-hydroxyethyl)-N-methylamino, (5-hydroxy-4-methyl-4H-1,2,4-triazol-3-yl)thio, (4-hydroxycyclohexyl)amino, 1,3,4-thiazol-2-ylthio, [4-hydroxy-2-cyclopenten-1-yl]amino, 3-hydroxy-1-pyrrolidinyl, 3-hydroxy-3-methyl-1-azetidinyl, 3-amino-1-pyrrolidinyl and (2-aminoethyl)thio.

Particularly preferred compounds of the invention include:
2-[[(2,3-difluorophenyl)methyl]thio]-6-[(2-hydroxyethyl)amino]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[(phenylmethyl)amino]-7(8H)-pteridinone;
2-[[(2,3-Difluorophenyl)methyl]thio-4-[[(1R)-2-hydroxy-1-methylethyl]amino]-6,7-pteridinedione;
6-amino-2-[[(2,3-difluorophenyl)methyl]thio]-4-[[(1*R*)-2-hydroxy-1-methylethyl]amino]-7(8*H*)-pteridinone;
2-[[2,3-difluorophenyl)methyl)thio]-4-[[(1R)-2-hydroxy-1-methylethyl]amino]-6-(1H-imidazol-1-yl)-7(8H)-pteridinone;
2-[[2,3-difluorophenyl)methyl)thio]-4-[[(1R)-2-hydroxy-1-methylethyl]amino]-6-[(1-methyl-1H-imidazol-2-yl)thio]-7(8H)-pteridinone;
2-[[2,3-difluorophenyl)methyl)thio]-4-[[(1R)-2-hydroxy-1-methylethyl]amino]-6-methoxy-7(8H)-pteridinone;
2-[[(2,3-difluorophenyl)methyl]thio]-4-[[(1R)-2-hydroxy-1-methylethyl]amino]-6-[(3-pyridinylmethyl)amino]-7(8H)-pteridinone;
2-[[(2,3-difluorophenyl)methyl]thio]-4-[[(1R)-2-hydroxy-1-methylethyl]amino]-6-[[(5-methyl-2-furanyl)methyl]amino]-7(8H)-pteridinone ;
2-[[(2,3-difluorophenyl)methyl]thio]-6-[(3R,5S)-3,5-dimethyl-1-piperazinyl]-4-[[(1R)-2-hydroxy-1-methylethyl]amino]-7(8H)-pteridinone ;
2-[[(2,3-difluorophenyl)methyl]thio]-4-[[(1R)-2-hydroxy-1-methylethyl]amino]-6-[methyl[(3-methyl-5-isoxazolyl)methyl]amino]-7(8H)-pteridinone;
2-[[(2,3-difluorophenyl)methyl]thio]-4-[[(1R)-2-hydroxy-1-methylethyl]amino]-6-[[2-(2-pyrimidinylamino)ethyl]amino]-7(8H)-pteridinone ;
2-[[(2,3-difluorophenyl)methyl]thio]-4-[[(1R)-2-hydroxy-1-methylethyl]amino]-6-(4-morpholinyl)-7(8H)-pteridinone;
2-[[(2,3-difluorophenyl)methyl]thio]-4-[[(1R)-2-hydroxy-1-methylethyl]amino]-6-([2-(4-morphohinyl)ethyl]amino]-7(8H)-pteridinone;
2-[[(2,3-difluorophenyl)methyl]thio]-4-[[(1R)-2-hydroxy-1-methylethyl]amino]-6-[(2-methoxyethyl)amino]-7(8H)-pteridinone;
2-[[(2,3-difluorophenyl)methyl]thio]-6-[(2-furanylmethyl)amino]-4-[[(1R)-2-hydroxy-1-methylethyl]amino]-7(8H)-pteridinone;
6-(1-azetidinyl)-2-[[(2,3-difluorophenyl)methyl]thio]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[[(5-methylpyrazinyl)methyl]amino]-7(8H)-pteridinone;
2-[[(2,3-difluorophenyl)methyl]thio]-6-[[2-(2-furanyl)ethyl]amino]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[[3-(4-morpholinyl)propyl]amino]-7(8H)-pteridinone;
2-[[(2,3-difluorophenyl)methyl]thio]-4-[[(1R)-2-hydroxy-1-methylethyl]amino]-6-[[(3-methyl-5-isoxazolyl)methyl]amino]-7(8H)-pteridinone;
2-[[(2,3-difluorophenyl)methyl]thio]-4-[[(1R)-2-hydroxy-1-methylethyl]amino]-6-[(3S)-3-hydroxy-1-pyrrolidinyl]-7(8H)-pteridinone;
2-[[(2,3-difluorophenyl)methyl]thio]-6-[(2-furanylmethyl)thio]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[(2-hydroxypropyl)amino]-7(8H)-pteridinone;
2-[[(2,3-difluorophenyl)methyl]thio]-6-[[2-(dimethylamino)ethyl]thio]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[[(2S)-2-hydroxypropyl]amino]-7(8H)-pteridinone;
2-[[(2,3-difluorophenyl)methyl]thio]-4-[[(1R)-2-hydroxy-1-methylethyl]amino]-6-[(3-hydroxypropyl)amino]-7(8H)-pteridinone;
2-[[(2,3-difluorophenyl)methyl]thio]-6-[(2-hydroxyethyl)methylamino]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[(5-hydroxy-4-methyl-4H-1,2,4-triazol-3-yl)thio]-7(8H)-pteridinone;
2-[[(2,3-difluorophenyl)methyl]thio]-6-[(4-hydroxycyclohexyl)amino]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-(1,3,4-thiadiazol-2-ylthio)-7(8H)-pteridinone;
2-[[(2,3-difluorophenyl)methyl]thio]-6-[[(1S,4R)-4-hydroxy-2-cyclopenten-1-yl]amino]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[(3R)-3-hydroxy-1-pyrrolidinyl]-7(8H)-pteridinone;
2-[[(2,3-difluorophenyl)methyl]thio]-6-(3-hydroxy-3-methyl-1-azetidinyl)-4-[[(1R)-2-hydroxy-1-methylethyl] amino]-7(8H)-pteridinone;
6-[(3S)-3-amino-1-pyrrolidinyl]-2-[[(2,3-difluorophenyl)methyl]thio]-4-[[(1R)-2-hydroxy-1-methylethyl]amino]-7(8H)-pteridinone; and
6-[(2-aminoethyl)thio]-2-[[(2,3-difluorophenyl)methyl]thio]-4-[[(1R)-2-hydroxy-1-methylethyl]amino]-7(8H)-pteridinone.

According to the invention there is also provided a process for the preparation of:-
(a) a compound of formula (I) where Y is NR²⁰R²¹ which comprises treatment of a compound of formula (IIA) where R¹, R² and R³ are as defined in formula (I) or are protected derivatives thereof and L is a leaving group such as bromo with an amine HNR²⁰R²¹, 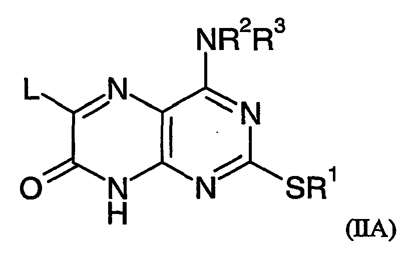
(b) a compound of formula (I) where Y is OR⁴ which comprises treatment of a compound of formula (IIA) where R¹, R² and R³ are as defined in formula (I) or are protected derivatives thereof and L is a leaving group such as bromo with an alcohol R⁴OH,
(c) a compound of formula (I) where Y is SR⁴ which comprises treatment of a compound of formula (IIA) where R¹, R² and R³ are as defined in formula (I) or are protected derivatives thereof and L is a leaving group such as bromo with a thiol R⁴SH,
(d) a compound of formula (I) where Y is NR⁵R⁶which comprises treatment of a compound of formula (IIA) where R¹, R² and R³ are as defined in formula (I) or are protected derivatives thereof and L is a leaving group such as bromo with an amine H NR⁵R⁶
(e) a compound of formula (I) where Y is a heteroaryl group which comprises treatment of a compound of formula (IIA) where R¹, R² and R³ are as defined in formula (I) or are protected derivatives thereof and L is a leaving group such as bromo with a heteroarene.
(f) a compound of formula (I) where Y is OH which comprises treatment of a compound of formula (IIB): 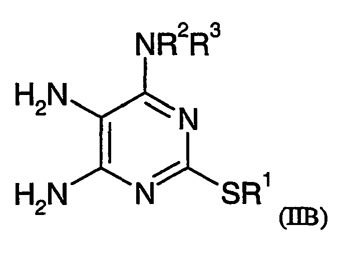 where R¹, R² and R³ are as defined in formula (I) or are protected derivatives thereof with diethyl oxalate, or
(g) a compound of formula (I) where Y is NH₂ which comprises treatment of a compound of formula (IIB) where R¹, R² and R³ are as defined in formula (I) or are protected derivatives thereof with iminomethoxy-acetic acid, methyl ester hydrochloride, and optionally thereafter process (a), (b), (c), (d) or (e) and in any order:
   - removing any protecting groups
   - forming a pharmaceutically acceptable salt, solvate or *in vivo* hydrolysable ester.

The reaction of compounds of formula (IIA) with an amine HNR²⁰R²¹ may be performed in a solvent such as N-methylpyrrolidinone at a temperature between 0°C and 150°C in the presence of a base such as *N,N-*diisopropylethylamine.

The reaction of compounds of formula (IIA) with an alcohol R⁴OH may be performed using the alcohol R⁴OH as solvent at a temperature between 0°C and 150°C in the presence of a base such as butyllithium.

The reaction of compounds of formula (IIA) with a thiol R⁴SH may be performed in a solvent such as DMSO at a temperature between 0°C and 150°C in the presence of a base such as potassium tert-butoxide.

The reaction of compounds of formula (IIA) with an amine HNR⁵R⁶ may be performed in a solvent such as N-methylpyrrolidinone at a temperature between 0°C and 150°C in the presence of a base such as *N,N-*diisopropylethylamine

The reaction of compounds of formula (IIA) with a heteroarene may be performed in a solvent such as DMSO at a temperature between 0°C and 100°C in the presence of a base such as potassium tert-butoxide.

The reaction of compounds of formula (IIB) with diethyl oxalate may be performed in the absence of solvent at a temperature between 50°C and 200°C.

The reaction of compounds of formula (IIB) with iminomethoxy-acetic acid, methyl ester hydrochloride may be performed in ethanol in the presence of base such as *N,N-*diisopropylethylamine at a temperature between 0°C and 150°C.

Compounds of formula (IIA) where R¹, R² and R³ are as defined in formula (I) and L is bromo may be prepared from compounds of formula (IIA) where R¹, R² and R³ are as defined above and L is hydrogen by treatment with bromine in a solvent such as acetonitrile at a temperature between 0°C and 100°C.

Compounds of formula (IIA) where R¹, R² and R³ are as defined in formula (I) and L is hydrogen may be prepared from compounds of formula (III) where R¹ is as defined above and X is a leaving group such as bromo by treatment with an amine HNR²R³. The reaction may be performed in a solvent such as N-methylpyrrolidinone at a temperature between 0°C and 150°C in the presence of a base such as *N,N*-diisopropylethylamine.

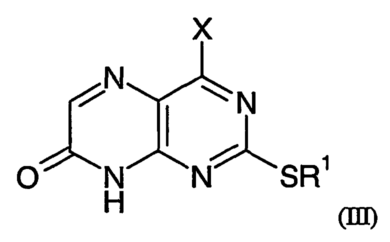

Compounds of formula (III) where R¹ is as defined in formula (I) and X is a leaving group such as bromo may be prepared by treating a compound of formula (III) where R¹ is as defined above and X is NH₂ with a diazotizing agent such as isoamyl nitrite in the presence of a halogenating agent such as bromoform. The reaction may be performed in a solvent such as DMSO at a temperature between 0°C and 150°C.

Compounds of formula (III) where R¹ is as defined in formula (I) and X is NH₂ may be prepared by treatment of a compound of formula (IV): 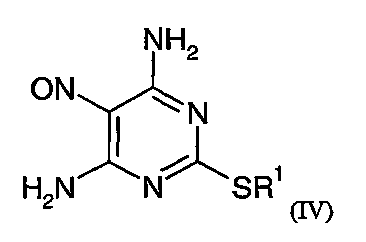 where R¹ is as defined above with triethyl phosphonoacetate in the presence of a base such as butyllithium. The reaction may be carried out in a solvent such as DMF at a temperature between 0°C and 100°C.

Compounds of formula (IV) where R¹ is as defined in formula (I) may be prepared by treating a compound of formula (V) where R¹ is as defined above with a nitrosating agent such as sodium nitrite. The reaction may be performed in a solvent such as aqueous acetic acid at a temperature between 0°C and 100°C.

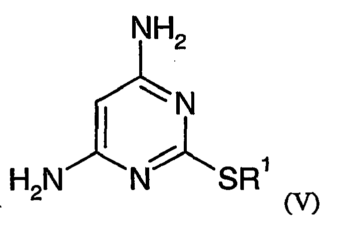

Compounds of formula (V) where R¹ is as defined in formula (I) may be prepared by treating a compound of formula (VI) with a compound of formula R¹X where R¹ is as defined above and X is a leaving group such as bromide in the presence of a base such as potassium tert-butoxide. The reaction may be performed in a solvent such as DMSO at room temperature.

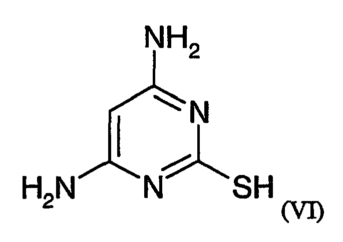

Compounds of formula (IIB) where R¹, R² and R³ are as defined in formula (I) may be prepared by treatment of compounds of formula (VII) where R¹, R² and R³ are as defined above with a reducing agent such as zinc. The reaction may be performed in a solvent such as ethanol at reflux.

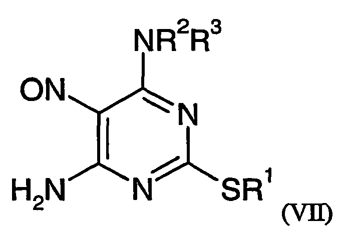

Compounds of formula (VII) where R¹, R² and R³ are as defined in formula (I) may be prepared by treatment of compounds of formula (VIII) where R¹, R² and R³ are as defined above with a nitrosating agent such as sodium nitrite in acetic acid. The reaction may be conveniently carried out at room temperature.

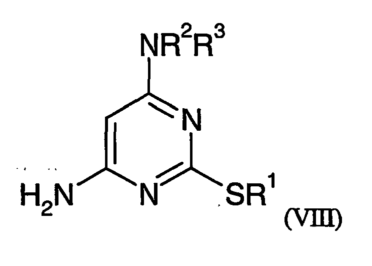

Compounds of formula (VIII) where R¹, R² and R³ are as defined in formula (I) may be prepared by treatment of compounds of formula (IX) where R¹ is as defined in formula (I) and L is a leaving group such as chloro with an amine HNR²R³. The reaction can be carried out in a solvent such as N-methylpyrrolidinone at elevated temperature, for example at between 50°C and 200°C.

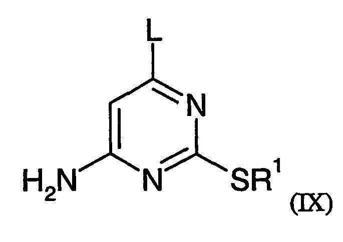

Compounds of formula (IX) where R¹ is as defined in formula (I) and L is a leaving group such as chloro may be prepared by treatment of compounds of formula (IX) where R¹ is as defined in formula (I) and L is hydroxy by treatment with a halogenating agent such as phosphorus oxychloride. The reaction may be carried out in the presence of a base such as 2-picoline at a temperature between 0°C and 150°C.

Compounds of formula (IX) where R¹ is as defined in formula (I) and L is hydroxy may be prepared from compounds of formula (X) by treatment with a compound of formula R¹X where R¹ is as defined above and X is a leaving group such as bromide. The reaction may be carried out in a solvent such as aqueous DMF using a base such as potassium hydroxide at room temperature.

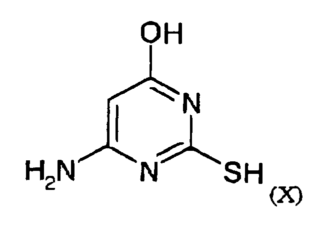

Compounds of formula (VI) and (X) are commercially available.

It will be appreciated by those skilled in the art that in the processes of the present invention certain functional groups such as hydroxyl or amino groups in the starting reagents or intermediate compounds may need to be protected by protecting groups. Thus, the preparation of the compounds of formula (I) may involve, at an appropriate stage, the removal of one or more protecting groups. The protection and deprotection of functional groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wuts, Wiley-Interscience (1991).

Novel intermediate compounds form a further aspect of the invention. In particular an intermediate of formula (IIA) is provided. L is a leaving group selected from hydroxy, bromo or chloro; R² is hydrogen; R³ is CH(CH₃)CH₂OH; and R¹ is benzyl substituted by two fluorine atoms.

The compounds of formula (I) above may be converted to a pharmaceutically acceptable salt or solvate thereof, preferably a basic addition salt such as sodium, potassium, calcium, aluminium, lithium, magnesium, zinc, henzathine, chloroprocaine, choline, diethanolamine, ethanolamine, ethyldiamine, meglume, tromethamine or procaine, or an acid addition salt such as a hydrochloride, hydrobromide, phosphate, acetate, fumarate, maleate, tartrate, citrate, oxalate, methanesulphonate or p-toluenesulphonate.

The compounds of formula (I) above may be converted to a pharmaceutically acceptable *in vivo* hydrolysable ester thereof. An *in vivo* hydrolysable ester of a compound of formula (I) that contains a carboxy or a hydroxy group is, for example a pharmaceutically acceptable ester which is hydrolysed in the human or animal body to produce the parent acid or alcohol. Such esters can be identified by administering, for example, intravenously to a test animal, the compound under test and subsequently examining the test animal's body fluid.

Suitable pharmaceutically acceptable esters for carboxy include C₁-C₆ alkoxymethyl esters for example methoxymethyl, C₁-C₆ alkanoyloxymethyl esters for example pivaloyloxymethyl, phthalidyl esters, C₃-C₈ cycloalkoxycarbonyloxyC₁-C₆alkyl esters for example 1-cyclohexylcarbonyloxyethyl; 1,3-dioxolen-2-onylmethyl esters for example 5-methyl-1,3-dioxolen-2-onylmethyl; and C₁-C₆ alkoxycarbonyloxyethyl esters for example 1-methoxycarbonyloxyethyl and may be formed at any carboxy group in the compounds of this invention.

Suitable pharmaceutically-acceptable esters for hydroxy include inorganic esters such as phosphate esters (including phosphoramidic cyclic esters) and α-acyloxyalkyl ethers and related compounds which as a result of the *in-vivo* hydrolysis of the ester breakdown to give the parent hydroxy group/s. Examples of α-acyloxyalkyl ethers include acetoxymethoxy and 2,2-dimethylpropionyloxymethoxy. A selection of in-vivo hydrolysable ester forming groups for hydroxy include C₁-C₁₀ alkanoyl, for example formyl, acetyl; benzoyl; phenylacetyl; substituted benzoyl and phenylacetyl, C₁-C₁₀ alkoxycarbonyl (to give alkyl carbonate esters), for example ethoxycarbonyl; di-(C₁-C₄)alkylcarbamoyl and *N*-(di-(C₁-C₄)alkylaminoethyl)-*N-*(C₁-C₄)alkylcarbamoyl (to give carbamates); di-(C₁-C₄)alkylaminoacetyl and carboxyacetyl. Examples of ring substituents on phenylacetyl and benzoyl include aminomethyl, (C₁-C₄)allcylaminomethyl and di-((C₁-C₄)alkyl)aminomethyl, and morpholino or piperazino linked from a ring nitrogen atom via a methylene linking group to the 3- or 4- position of the benzoyl ring. Other interesting in-vivo hydrolysable esters include, for example, R^{A}C(O)O(C₁-C₆)alkyl-CO-, wherein R^{A} is for example, benzyloxy-(C₁-C₄)alkyl, or phenyl). Suitable substituents on a phenyl group in such esters include, for example, 4-(C₁-C₄)piperazino-(C₁-C₄)alkyl, piperazino-(C₁-C₄)alkyl and morpholino-(C₁-C₄)alkyl.

The compounds of formula (I) have activity as pharmaceuticals, in particular as modulators of chemokine receptor (especially CXCR2) activity, and may be used in the treatment (therapeutic or prophylactic) of conditions/diseases in human and non-human animals which are exacerbated or caused by excessive or unregulated production of chemokines. Examples of such conditions/diseases include:
(1) **(the respiratory tract)** obstructive airways diseases including chronic obstructive pulmonary disease (COPD); asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) **(bone and joints)** rheumatoid arthritis, seronegative spondyloarthropathies (including ankylosing spondylitis, psoriatic arthritis and Reiter's disease), Behchet's disease, Sjogren's syndrome and systemic sclerosis;
(3) **(skin)** psoriasis, atopical dermatitis, contact dermatitis and other eczematous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) **(gastrointestinal tract)** Coeliac disease, proctitis, eosinopilic gastro-enteritis, mastocytosis, Crohn's disease, ulcerative colitis, food-related allergies which have effects remote from the gut, e.g., migraine, rhinitis and eczema;
(5) **(central and peripheral nervous system)** Neurodegenerative diseases and dementia disorders, e.g. Alzheimer's disease, amyotrophic lateral sclerosis and other motor neuron diseases, Creutzfeldt-Jacob's disease and other prion diseases, HIV encephalopathy (AIDS dementia complex), Huntington's disease, frontotemporal dementia, Lewy body dementia and vascular dementia; polyneuropathies, e.g. Guillain-Barré syndrome, chronic inflammatory demyelinating polyradiculoneuropathy, multifocal motor neuropathy, plexopathies; CNS demyelination, e.g. multiple sclerosis, acute disseminated/haemorrhagic encephalomyelitis, and subacute sclerosing panencephalitis; neuromuscular disorders, e.g. myasthenia gravis and Lambert-Eaton syndrome; spinal diorders, e.g. tropical spastic paraparesis, and stiff-man syndrome: paraneoplastic syndromes, e.g. cerebellar degeneration and encephalomyelitis; CNS trauma; migraine; and stroke;
(6) **(other tissues and systemic disease)** atherosclerosis, Acquired Immunodeficiency Syndrome (AIDS), lupus erythematosus, systemic lupus, erythematosus, Hashimoto's thyroiditis, type I diabetes, nephrotic syndrome, eosinophilia fascitis, hyper IgE syndrome, lepromatous leprosy, and idiopathic thrombocytopenia pupura; post-operative adhesions, and sepsis;
(7) **(allograft rejection)** acute and chronic following, for example, transplantation of kidney, heart, liver, lung, bone marrow, skin and cornea; and chronic graft versus host disease;
(8) Cancers, especially non-small cell lung cancer (NSCLC), malignant melanoma, prostate cancer and squamous sarcoma, and tumour metastasis, non melanoma skin cancer and chemoprevention of metastases;
(9) Diseases in which angiogenesis is associated with raised CXCR2 chemokine levels (e.g. NSCLC, diabetic retinopathy);
(10) Cystic fibrosis;
(11) Burn wounds & chronic skin ulcers;
(12) Reproductive Diseases (e.g. Disorders of ovulation, menstruation and implantation, Pre-term labour, Endometriosis);
(13) Re-perfusion injury in the heart, brain, peripheral limbs and other organs, inhibition of atherosclerosis.

Thus, the present invention provides a compound of formula (I), or a pharmaceutically-acceptable salt or solvate thereof, as hereinbefore defined for use in therapy.

The present invention also provides a pharmaceutically acceptable *in vivo* hydrolysable ester of a compound of formula (I), as hereinbefore defined for use in therapy.

Preferably the compounds of the invention are used to treat diseases in which the chemokine receptor belongs to the CXC chemokine receptor subfamily, more preferably the target chemokine receptor is the CXCR2 receptor.

Particular conditions which can be treated with the compounds of the invention are psorisis, rheumatoid arthritis, diseases in which angiogenesis is associated with raised CXCR2 chemokine levels, and respiratory disease such as COPD. It is preferred that the compounds of the invention are used to treat rheumatoid arthritis. The compounds of the invention may also be used to treat COPD.

As a further aspect of the present invention, certain compounds of formula (I) may have utility as antagonists of the CX3CR1 receptor. Such compounds are expected to be particularly useful in the treatment of disorders within the central and peripheral nervous system and other conditions characterized by an activation of microglia and/or infiltration of leukocytes (e.g. stroke/ischemia and head trauma).

In another aspect, the present invention provides the use of a compound of formula (I), or a pharmaceutically acceptable salt or solvate thereof, as hereinbefore defined as a medicament.

In another aspect, the present invention provides the use of a pharmaceutically acceptable *in vivo* hydrolysable ester of a compound of formula (I), as hereinbefore defined as a medicament.

In a further aspect, the present invention provides the use of a compound of formula (I), or a pharmaceutically acceptable salt or solvate thereof, as hereinbefore defined in the manufacture of a medicament for use in therapy.

The present invention also provides the use of a pharmaceutically acceptable *in vivo* hydrolysable ester of a compound of formula (I), as hereinbefore defined in the manufacture of a medicament for use in therapy.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of chemokine receptor activity is beneficial.

The present invention also provides the use of a pharmaceutically acceptable *in vivo* hydrolysable ester of a compound of formula (I), as hereinbefore defined in the manufacture of a medicament for the treatment of human diseases or conditions in which modulation of chemokine receptor activity is beneficial.

In the context of the present specification, the term "therapy" also includes "prophylaxis" unless there are specific indications to the contrary. The terms "therapeutic" and "therapeutically" should be construed accordingly.

We disclose a method of treating a chemokine mediated disease wherein the chemokine binds to a chemokine (especially CXCR2) receptor, which comprises administering to a patient a therapeutically effective amount of a compound of formula (I), or a pharmaceutically acceptable salt or solvate thereof, as hereinbefore defined.

We disclose a method of treating a chemokine mediated disease wherein the chemokine binds to a chemokine (especially CXCR2) receptor, which comprises administering to a patient a therapeutically effective amount of a pharmaceutically acceptable *in vivo* hydrolysable ester of a compound of formula (I), as hereinbefore defined.

We disclose a method of treating an inflammatory disease, especially psoriasis, in a patient suffering from, or at risk of, said disease, which comprises administering to the patient a therapeutically effective amount of a compound of formula (I), or a pharmaceutically acceptable salt or solvate thereof, as hereinbefore defined.

We disclose a method of treating an inflammatory disease, especially psoriasis, in a patient suffering from, or at risk of, said disease, which comprises administering to the patient a therapeutically effective amount of a pharmaceutically acceptable *in vivo* hydrolysable ester of a compound of formula (I), as hereinbefore defined.

We disclose a method of treating an inflammatory disease, especially rheumatoid arthritis, COPD and psoriasis, in a patient suffering from, or at risk of, said disease, which comprises administering to the patient a therapeutically effective amount of a compound of formula (I), or a pharmaceutically acceptable salt, solvate or *in vivo* hydrolysable ester thereof, as hereinbefore defined. Preferably the method of treating rheumatoid arthritis is provided. Also provided is a method of treating COPD.

For the above-mentioned therapeutic uses the dosage administered will, of course, vary with the compound employed, the mode of administration, the treatment desired and the disorder indicated.

The compounds of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The present invention further provides a pharmaceutical composition comprising a pharmaceutically acceptable *in vivo* hydrolysable ester of a compound of formula (I), as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The invention further provides a process for the preparation of a pharmaceutical composition of the invention which comprises mixing a pharmaceutically acceptable *in vivo* hydrolysable ester of a compound of formula (I), as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compounds of the invention are administered orally.

The invention will now be further illustrated by reference to the following examples. In the examples the Nuclear Magnetic Resonance (NMR) spectra were measured on a Varian Unity Inova 300 or 400 MHz spectrometer and the Mass Spectrometry (MS) spectra measured on a Finnigan Mat SSQ7000 or Micromass Platform spectrometer or Agilent MSD spectrometer. Where necessary, the reactions were performed under an inert atmosphere of either nitrogen or argon. Chromatography was generally performed using Matrex Silica 60^{®} (35-70 micron) or Prolabo Silica gel 60^{®} (35-70 micron) suitable for flash silica gel chromatography. High pressure liquid chromatography purification was performed using a Waters Micromass LCZ with a Waters 600 pump controller, Waters 2487 detector and Gilson FC024 fraction collector or a Waters Delta Prep 4000 or a Gilson Auto-Purification System. The abbreviations m.p. and DMSO used in the examples stand for melting point and dimethyl sulphoxide respectively.

### EXAMPLES

### Example 1

### 2-[[(2,3-difluorophenyl)methyl]thio]-6-[(2-hydrogyethyl)amino]-4-[[(1R)-2-hydroxy-1-methylethyl]amino]-7(8H)-pteridinone

### a) 2-[[(2,3-Difluorophenyl)methyl]thio]- 4,6-pyrimidinediamine

4,6-diamino-2-pyrimidinethiol (7.3g) was dissolved in DMSO (100ml) at room temperature under an atmosphere of nitrogen. Potassium tert-butoxide (1M in THF, 48.3ml) was added followed by 2,3-difluorobenzyl-bromide (10.0g). The mixture was stirred for 2 hours at room temperature. The reaction mixture was then partitioned between ethyl acetate and ammonium chloride. The organic phase was washed with ammonium chloride (3x) and brine, then dried over magnesium sulphate and evaporated to give the subtitled product as a white solid (12.2g)
MS: ADCI (+ve) 269 (M+1)

### b) 2-[[(2,3-Difluorophenyl)methyl]thio]-5-nitroso-4,6-pyrimidinediamine

The product of Example 1, step (a) (2.5g) was dissolved in acetic acid (150ml) and the solution cooled to 5°C. A solution of sodium nitrite (625mg) in water (50ml) was added dropwise resulting in a dark blue colouration. The reaction was stirred at room temperature for 30 minutes during which time a pink solid precipitated from solution. This was isolated by filtration and washed with water, then dried at 50°C to give the sub-titled product as a blue solid (4.14g)
MS: ADCI (+ve) 298 (M+1)
¹H NMR: δ (DMSO) 4.44 (s,2H), 7.13-7.54 (m,3H), 8.13 (s,1H), 8.51 (s,1H), 9.10 (s, 1H), 10.18 (s,1H).

### c) 4-amino-2-[[(2,3-difluorophenyl)methyl]thio]-7(8H)-pteridinone

To a solution of triethyl phosphonoacetate (15.0g) in tetrahydrofuran (60ml) cooled in an ice bath was added butyllithium (2.5M in hexanes, 25.6ml) at a rate such that the internal temperature was maintained below 30°C. To this mixture was then added a solution of the product of Example 1, step (b) (10.0g) in *N,N-*dimethylformamide (60ml). The reaction mixture was heated at reflux for 1 hour, then cooled to room temperature and quenched with acetic acid (6ml). The solid thus precipitated was isolated by filtration, washed with water, ethanol and diethyl ether, and dried over P₂O₅ at 50°C to give the sub-titled product as a pale green solid (9.3g).
MS: ADCI (+ve) 322 (M+1)
¹H NMR: δ (DMSO) 4.18 (s,2H), 7.11-7.58 (m,3H), 7.84 (s,1H), 12.69 (bs,1H)

### d) 4-bromo-2-[[(2,3-difluorophenyl)methyl]thio] 7(8H)-pteridinone

The product of Example 1, step (c) (0.5g) was suspended in DMSO (10ml) and bromoform (10ml) and the mixture was heated to 125°C. Isoamylnitrite (2ml) was added and the mixture was stirred at 125°C for 5 minutes before being cooled in an ice bath. Solvent was removed by evaporation under high vacuum and the residue suspended in dichloromethane (100ml). This suspension was washed with saturated aqueous ammonium chloride (50ml) and then filtered through a plug of celite. The filtrate was evaporated and purified by column chromatography, eluting with 10% ethyl acetate in dichloromethane to give the subtitled compound as a white solid (0.22g).
MS: ADCI (+ve) 386 (M+1)
¹H NMR: δ (DMSO) 4.47 (s,2H), 7.13-7.55 (m,3H), 8.14 (s,1H), 13.33 (bs,1H)

### e) 2-[[(2,3-difluorophenyl)methyl]thio]-4-[[(1R)-2-hydroxy-1-methylethyl]amino]-7(8H)-pteridinone

The product of Example 1, step (d) (8.7g) was dissolved in N-methylpyrrolidinone (40ml) and Hunigs base (7.9ml) was added followed by D-alaninol (2.7ml). The mixture was stirred at 100°C for 15mins. The cooled solution was poured onto water, (11), and acidified with dilute hydrochloric acid. The solid which separated was collected, washed with water and air dried. Crystallisation from acetonitrile afforded the title compound as a pale yellow solid (7.4g).
m.p. 215-217°C
MS: APCI (+ve) 380 (M+H, 100%)
¹H NMR: δ (DMSO) 1.14 (d, 3H), 3.48 (m, 2H), 4.31 (m, 1H), 4.45 (dd, 2H) 4.82 (t, 1H) 7.15 (m, 1H), 7.33 (m, 1H), 7.47 (t, 1H), 7.76 (d, 1H), 7.83 (d,1H), 12.70 (s,1H).

### (f) 6-bromo-2-[[(2,3-difluorophenyl)methyl]thio]-4-[[(1R)-2-hydroxy-1-methylethyl]amino]-7(8H)-pteridinone

The product of Example 1, step (e) (5.0g) was suspended in acetonitrile (200ml) and bromine (1.2ml) added. The reaction was stirred at room temperature for 2 hours, then evaporated to dryness. The crude product was purified by column chromatography on silica gel, eluting with 2% methanol in dichloromethane to give the subtitled compound as a pale yellow solid (1.7g).
MS: APCI (+ve) 458/460 (M+H, 100%)
¹H NMR: δ (DMSO) 7.76 (d, 1H), 7.47 (m, 1H), 7.33 (m, 1H), 7.16 (m, 1H), 4.85 (t, 1H), 4.45 (q, 1H), 4.34 (m, 1H), 3.54 (m, 1H), 3.45 (m, 1H), 1.15 (d, 3H).

### (g) 2-[[(2,3-difluorophenyl)methyl]thio]-6-[(2-hydroxyethyl)amino]-4-[[(1R)-2-hydroxy-1-methylethyl]amino]-7(8H)-pteridinone

The product of Example 1, step (f) (50mg), ethanolamine (13mg) and *N,N-*diisopropylethylamine (38ul) were dissolved in N-methylpyrrolidinone (2ml) and heated to 100°C for 2 hours. The cooled reaction mixture was diluted with ethyl acetate and washed 5x with saturated aqueous ammonium chloride. The organic phase was dried over magnesium sulphate, filtered and evaporated. The crude product was purified by column chromatography on silica gel, eluting with 10% methanol in dichloromethane, followed by trituration with methanol to give the titled compound as a white solid (10mg).
m.p. 197-199°C
MS: APCI (+ve) 439 (M+H, 100%)
¹H NMR: δ (DMSO) 12.45 (br s, 1H), 7.45 (m, 1H), 7.29 (m, 2H), 7.14 (m, 1H), 6.66 (d, 1H), 4.87 (t, 1H), 4.77 (t, 1H), 4.40 (s, 2H), 4.22 (m, 1H), 3.59 (m, 2H), 3.49 (m, 4H), 1.16 (d, 3H).

### Example 2

### 2-[[(2,3-difluorophenyl)methyl]thio]-4-[[(1R)-2-hydroxy-1-methylethyl]amino]-6-[(phenylmethyl)amino]-7(8H)-pteridinone

The product of Example 1, step (f) (50mg), benzylamine (35mg) and *N,N-*diisopropylethylamine (38ul) were dissolved in N-methylpyrrolidinone (2ml) and heated to 100°C for 2 hours. The cooled reaction mixture was diluted with ethyl acetate and washed 5x with saturated aqueous ammonium chloride. The organic phase was dried over magnesium sulphate, filtered and evaporated. The crude product was purified by column chromatography on silica gel, eluting with 2% methanol in dichloromethane to give the titled compound as a pale yellow solid (24mg).
m.p. 80-100°C
MS: APCI (+ve) 485 (M+H, 100%)
¹H NMR: δ (DMSO) 9.10 (br s, 1H), 7.35 (m, 6H), 7.02 (m, 2H), 6.57 (br t, 1H), 6.07 (br d, 1H), 4.62 (m, 2H), 4.38 (s, 2H), 4.33 (m, 1H), 3.76 (m, 1H), 3.64 (m, 1H), 2.62 (m, 1H), 1.28 (d, 3H).

### Example 3

### 2-[[(2,3-Difluorophenyl)methyl]thio-4-[[(1R)-2-hydroxy-1-methylethyl]amino]-6,7-pteridinedione

### (a) 6-amino-2-[[(2,3-difluorophenyl)methyl]thio]-4-pyrimidinol,

To a stirred suspension of 4-amino-6-hydroxy-2-mercaptopyrimidine monohydrate (50g) in DMF (600ml) was added potassium hydroxide (20.2g), water (100ml) and 2,3-difluorobenzyl bromide (64.2g). The reaction was stirred at room temperature for 30min. The mixture was poured into water (41) and the resulting precipitate isolated by filtration, washing with isopropanol, to afford the subtitled compound as a white solid (73.7g).
m.p. 218-221°C
MS: APCI+ve 270 (M+H, 100%)
¹H NMR: δ (DMSO) 7.37 (1H, m), 7.35 (1H, m), 7.16 (1H, m), 6.57 (3H, m), 4.99 (1H, br s), 4.39 (2H, s).

### (b) 6-chloro-2-[[(2,3-difluorophenyl)methyl]thiol-4-pyrimidinamine

The product of Example 3, step (a) (73g) was added to a solution of 2-picoline (40ml) in POCl₃ (300ml) and the mixture refluxed for 24hr. The reaction mixture was concentrated *in vacuo* to half its original volume and poured onto ice and then neutralized with ammonia, forming a brown solid. This mixture was refluxed for 1hr, and the precipitate isolated by filtration, washing with water. The crude product was purified by silica gel chromatography, eluting with dichloromethane to afford the subtitled compound as a white solid (31.7g).
MS: APCI (+ve) 288/290 (M+H, 100 %)
¹HNMR: δ (DMSO) 7.33 (5H, m), 7.12 (1H, m), 4.36 (2H, s).

### (c) (2R)-2-[[6-amino-2-[[(2,3-difluorophenyl)methyl]thio]-4-pyrimidinyl]amino]-1-propanol

A solution of the product from Example 3, step (b) (22g), *N,N-*diisopropylethylamine (110ml) and D-alaninol (23g) in NMP (150ml) was heated at 160°C for 48h. The reaction mixture was allowed to cool to room temperature and poured into aqueous ammonium chloride (1.21). The resultant white precipitate was purified by silica gel chromatography, eluting first with 3:2 DCM:ethyl acetate and then 3:1 DCM:methanol, giving the subtitled product as a pink solid (22.6 g).
MS: APCI (+ve) 327 (M+H, 100 %)
¹H NMR: δ (DMSO) 7.38 (1H, m), 7.29 (1H, m), 7.10 (1H, m), 6.40 (1H, d), 6.13 (2H, bs), 5.15 (1H, s), 4.66 (1H, t), 4.31 (2H, t), 4.02 (1H, bs), 3.39 (1H, m), 3.25 (1H, m), 1.05 (3H, d).

### (d) (2R)-2-[[6-amino-2-[[(2,3-difluorophenyl)methyl]thio]-5-nitroso-4-pyrimidinyl]amino]-1-propanol

To a stirred solution of the product from Example 3, step (c) (22g) in acetic acid (300ml) at room temperature was added a solution of sodium nitrite (4.8g) in water (30ml). The reaction was stirred at 0°C for 30min, and the resultant purple precipitate isolated by filtration, washing with water, to give the subtitled compound as a dark blue solid (37g, not completely dry).
MS: APCI (+ve) 356 (M+H, 100 %)

### (e) (2R)-2-[[5,6-diamino-2-[[(2,3-difluorophenyl)methyl]thio]-4-pyrimidinyl]amino]-1-propanol

To a solution of acetic acid (10ml) in boiling ethanol (300ml) was added zinc dust (15g) and the product of Example 3, step (d) (10g). The reaction was heated at reflux for 10 mins, cooled, filtered through celite and the filtrate evaporated. The crude product was triturated with water, filtered and dried *in vacuo* to to give the subtitled product as a cream solid (9.3g).
MS: APCI (+ve) 342 (M+H, 100 %)
¹H NMR: δ (DMSO) 7.34 (1H, m), 7.27 (1H, m), 7.12 (1H, m), 5.72 (2H, b s), 5.58 (1H,d), 4.65 (1H, t), 4.30 (2H, d), 4.04 (1H, m), 3.54 (2H, bs), 3.44 (1H, m), 3.29 (1H, m), 1.09 (3H, d).

### (f) 2-[[(2,3-Difluorophenyl)methyl]thio-4-[[(1R)-2-hydroxy-1-methylethyllamino]-6,7-pteridinedione

The product of Example 3, step (e) (0.30g) and diethyloxalate were heated at 160°C for 30 mins. The mixture was concentrated *in vacuo*. Purification by flash chromatography over silica using dichloromethane/methanol (9:1) as eluant afforded the title compound (0.045g).
m.p. 243-246°C
MS: APCI 396 (M+H, 100%)
¹H NMR: δ (DMSO) 1.12 (d, 3H), 3.43 (m, 2H), 4.14 (m, 1H), 4.38 (q, 2H), 6.79 (d, 1H), 7.13 (m, 1H), 7.30 (m, 1H), 7.45 (t, 1H).

### Example 4

### 6-amino-2-[[(2,3-difluorophenyl)methyl]thio]-4-[[(1R)-2-hydroxy-1-methylethyl]amino]-7(8H)-pteridinone

To a solution of the product from Example 3, step (e) (190 mg) in ethanol (30 ml) was added iminomethoxy-acetic acid, methyl ester hydrochloride (85mg) (J. Chem. Soc., Perkin 1, 1999, 1783-93) followed by *N,N*-diisopropylethylamine (70ul) and the mixture heated under reflux for 24 hours. The mixture was evaporated to dryness and purified by silica chromatography (ethyl acetate) to give the title compound (55mg).
MS APCI+ve 395 (M+H, 100%)
¹H NMR: δ (DMSO) 12.45 (bs, 1H), 7.45 (t, 1H), 7.31 (m, 1H), 7.27 (m, 1H), 7.06 (bst, 2H), 6.49 (d, 1H), 4.91 (t, 1H), 4.36 (ab, 2H), 4.17 (m, 1H), 3.47 (t, 2H), 1.13 (d, 3H).

### Example 5

### 2-[[2,3-Difluorophenyl)methyl)thio]-4-[[(1R)-2-hydroxy-1-methylethyl]amino]-6-(1H-imidazol-1-yl)-7(8H)-pteridinone

The product of Example 1, step (f) (250mg), imidazole (445mg) and potassium tert-butoxide (5.5ml, 1M solution in THF) were dissolved in DMSO (10ml) and the mixture heated to 100°C for 1 hour. The cooled reaction mixture was diluted with ethyl acetate and washed 2x with saturated aqueous ammonium chloride and 2x with water. The organic phase was dried over magnesium sulphate, filtered and evaporated to give a yellow solid, which was recrystallised twice from acetonitrile/methanol to give the title compound (30mg).
MS APCI+ve 446 (M+H, 100%)
¹H NMR: δ (DMSO) 13.19 (s, 1H), 8.95 (s, 1H), 8.38 (s, 1H), 7.71 (d, 1H), 7.49 (m, 1H), 7.34 (m, 1H), 7.16 (m, 1H), 7.11 (s,1H), 4.85 (t, 1H), 4.46 (ab, 2H), 4.30 (m, 1H), 3.50 (m, 2H), 1.19 (d, 3H).

### Example 6

### 2-[[2,3-Difluorophenyl)methyl)thio]-4-[[(1R)-2-hydroxy-1-methylethyl]amino]-6-[(1-methyl-M-imidazol-2-yl)thio]-7(8H)-pteridinone

The product of Example 1, step (f) (250mg), 1-methyl-1H imidazole-2-thiol (375mg) and butyllithium (0.6ml, 2.5M solution in hexanes) were dissolved in N-methylpyrrolidinone (10ml) and the mixture heated to 100°C for 2 hours. The cooled reaction mixture was diluted with ethyl acetate and washed 4x with saturated aqueous ammonium chloride. The organic phase was dried over magnesium sulphate, filtered and evaporated. The crude product was purified by silica chromatography (20:1 dichloromethane:methanol) to give the title compound (95mg).
m.p. 216-217°C
MS APCI+ve 492 (M+H, 100%)
¹H NMR: δ (DMSO) 13.00 (s, 1H), 7.52 (s, 1H), 7.42 (t, 1H), 7.30 (m, 1H), 7.13 (m, 2H), 5.97 (d, 1H), 4.90 (br s, 1H), 4.42 (s, 2H), 4.05 (m,1H), 3.59 (s, 3H), 3.38 (m, 2H), 1.06 (d, 3H).

### Example 7

### 2-[[2,3-Difluorophenyl)methyl)thio]-4-[[(1R)-2-hydroxy-1-methylethyl]amino]-6-methoxy-7(8H)-pteridinone

The product of Example 1, step (f) (250mg) was dissolved in methanol (10ml) and butyllithium (0.6ml, 2.5M solution in hexanes) was added. The mixture was heated at reflux for 48 hours. The cooled reaction mixture was evaporated and purified by silica chromatography (20:1 dichloromethane:methanol) followed by recrystallisation from methanol to give the title compound (15mg).
MS APCI+ve 410 (M+H, 100%)
¹H NMR: δ (DMSO) 7.46 (t, 1H), 7.32 (q, 1H), 7.13 (m, 1H), 6.96 (br d, 1H), 4.86 (t, 1H), 4.42 (ab, 2H), 4.28 (m, 1H), 3.97 (s, 3H), 3.47 (m, 2H), 1.16 (d, 3H).

### Examples 8 to 36

Examples 8 to 36 were prepared by the method of Example 1 step (g) by reaction of the product of Example 1, step (f) with the appropriate amine or thiol. The product purified by either (a) column chromatography on silica gel, eluting with 10% methanol in dichloromethane, followed by trituration with methanol or (b) reverse phase chromatography using a Waters Xterra column with acetonitrile and 0.2% 0.880 NH₄OH solution as buffer to give the products as a solid as shown in table 1.

**Table 1:**

| **Example Number** | **Compound Name** | **MS: APCI (+ve)** |
|---|---|---|
| **8** | 2-[[(2,3-difluorophenyl)methyl]thio]-4-[[(1R)-2-hydroxy-1-methylethyl]amino]-6-[(3-pyridinylmethyl)amino]-7(8H)-pteridinone | 486 (M+H, 100%) |
| **9** | 2-[[(2,3-difluorophenyl)methyl]thio]-4-[[(1R)-2-hydroxy-1-methylethyl]amino]-6-[[(5-methyl-2- | 489 (M+H, |
| | furanyl)methyl]amino]-7(8H)-pteridinone | 100%) |
| **10** | 2-[[(2,3-difluorophenyl)methyl]thio]-6-[(3R,5S)-3,5-dimethyl-1-piperazinyl]-4-[[(1R)-2-hydroxy-1-methylethyl]amino]-7(8H)-pteridinone | 492 (M+H, 100%) |
| **11** | 2-[[(2,3-difluorophenyl)methyl]thio]-4-[[(1R)-2-hydroxy-1-methylethyl]amino]-6-[methyl[(3-methyl-5-isoxazolyl)methyl]amino]-7(8H)-pteridinone | 446 (M+H, 100%) |
| **12** | 2-[[(2,3-difluorophenyl)methyl]thio]-4-[[(lR)-2-hydroxy-1-methylethyl]amino]-6-[[2-(2-pyrimidinylamino)ethyl]amino]-7(8H)-pteridinone | 516 (M+H, 100%) |
| **13** | 2-[[(2,3-difluorophenyl)methyl]thio]-4-[[(1R)-2-hydroxy-1-methylethyl]amino]-6-(4-morpholinyl)-7(8H)-pteridinone | 465 (M+H, 100%) |
| **14** | 2-[[(2,3-difluorophenyl)methyl]thio]-4-[[(lR)-2-hydroxy-1-methylethyl]amino]-6-[[2-(4-morpholinyl)ethyl]amino]-7(8H)-pteridinone | 508 (M+H, 100%) |
| **15** | 2-[[(2,3-difluorophenyl)methyl]thio]-4-[[(lR)-2-hydroxy-1-methylethyl]amino]-6-[(2-methoxyethyl)amino]-7(8H)-pteridinone | 453 (M+H, 100%) |
| **16** | 2-[[(2,3-difluorophenyl)methyl]thio]-6-[(2-furanylmethyl)amino]-4-[[(1R)-2-hydroxy-1-methylethyl]amino]-7(8H)-pteridinone | 475 (M+H, 100%) |
| **17** | 6-(1-azetidinyl)-2-[[(2,3-difluorophenyl)methyl]thio]-4-[[(1R)-2-hydroxy-1-methylethyl]amino]-7(8H)-pteridinone | 435 (M+H, |
| | | 100%) |
| **18** | 2-[[(2,3-difluorophenyl)methyl]thio]-4-[[(1R)-2-hydroxy-1-methylethyl]amino]-6-[[(5-methylpyrazinyl)methyl]amino]-7(8H)-pteridinone | 501 (M+H, 100%) |
| **19** | 2-[[(2,3-difluorophenyl)methyl]thio]-6-[[2-(2-furanyl)ethyl]amino]-4-[[(1R)-2-hydroxy-1-methylethyl]amino]-7(8H)-pteridinone | 489 (M+H, 100%) |
| **20** | 2-[[(2,3-difluorophenyl)methyl]thio]-4-[[(1R)-2-hydroxy-1-methylethyl]amino]-6-[[3-(4-morphohnyl)propyl]amino]-7(8H)-pteridinone | 522 (M+H, 100%) |
| **21** | 2-[[(2,3-difluorophenyl)methyl]flio]-4-[[(1R)-2-hydroxy-1-methylethyl]amino]-6-[[(3-methyl-5-isoxazolyl)methyl]amino]-7(8H)-pteridinone | 490 (M+H, 100%) |
| **22** | 2-[[(2,3-difluorophenyl)methyl]thio]-4-[[(1R)-2-hydroxy-1-methylethyl]amino]-6-[(3S)-3-hydroxy-1-pyrrolidinyl]-7(8H)-pteridinone | 465 (M+H, 100%) |
| **23** | 2-[[(2,3-difluorophenyl)methyl]thio]-6-[(2-furanylmethyl)thio]-4-[[(1R)-2-hydroxy-1-methylethyl]amino]-7(8H)-pteridinone | 492 (M+H, 100%) |
| **24** | 2-[[(2,3-difluorophenyl)methyl]thio]-4-[[(1R)-2-hydroxy-1-methylethyl]amino]-6-[(2-hydroxypropyl)amino]-7(8H)-pteridinone | 453 (M+H, 100%) |
| **25** | 2-[[(2,3-difluorophenyl)methyl]thio]-6-[[2-(dimethylamino)ethyl]thio]-4-[[(1R)-2-hydroxy-1-methylethyl]amino]-7(8H)-pteridinone | 483 (M+H, 100%) |
| **26** | 2-[[(2,3-difluorophenyl)methyl]thio]-4-[[(lR)-2-hydroxy-1-methylethyl]amino]-6-[[(2S)-2-hydroxypropyl]amino]- | 453 (M+H, |
| | 7(8H)-pteridinone | 100%) |
| **27** | 2-[[(2,3-difluorophenyl)methyl]thio]-4-[[(1R)-2-hydroxy-1-methylethyl]amino]-6-[(3-hydroxypropyl)amino]-7(8H)-pteridinone | 453 (M+H, 100%) |
| **28** | 2-[[(2,3-difluorophenyl)methyl]thio]-6-[(2-hydroxyethyl)methylamino]-4-[[(1R)-2-hydroxy-1-methylethyl]amino]-7(8H)-pteridinone | 453 (M+H, 100%) |
| **29** | 2-[[(2,3-difluorophenyl)methyl]thio]-4-[[(lR)-2-hydroxy-1-methylethyl]amino]-6-[(5-hydroxy-4-methyl-4H-1,2,4-triazol-3-yl)thio]-7(8H)-pteridinone | 509 (M+H, 100%) |
| **30** | 2-[[(2,3-difluorophenyl)methyl]thio]-6-[(4-hydroxycyclohexyl)amino]-4-[[(1R)-2-hydroxy-1-methylethyl]amino]-7(8H)-pteridinone | 493 (M+H, 100%) |
| **31** | 2-[[(2,3-difluorophenyl)methyl]thio]-4-[[(lR)-2-hydroxy-1-methylethyl]amino]-6-(1,3,4-thiadiazol-2-ylthio)-7(8H)-pteridinone | 496 (M+H, 100%) |
| **32** | 2-[[(2,3-difluorophenyl)methyl]thio]-6-[[(lS,4R)-4-hydroxy-2-cyclopenten-1-yl]amino]-4-[[(1R)-2-hydroxy-1-methylethyl]amino]-7(8H)-pteridinone | 477 (M+H, 100%) |
| **33** | 2-[[(2,3-difluorophenyl)methyl]thio]-4-[[(1R)-2-hydroxy-1-methylethyl]amino]-6-[(3R)-3-hydroxy-1-pyrrolidinyl]-7(8H)-pteridinone | 465 (M+H, 100%) |
| | | |
| **34** | 2-[[(2,3-difluorophenyl)methyl]thio]-6-(3-hydroxy-3-methyl-1-azetidinyl)-4-[[(1R)-2-hydroxy-1-methylethyl]amino]-7(8H)-pteridinone | 465 (M+H, 100%) |
| **35** | 6-[(3S)-3-amino-1-pyrrolidinyl]-2-[[(2,3-difluorophenyl)methyl]thio]-4-[[(1R)-2-hydroxy-1-methylethyl]amino]-7(8H)-pteridinone | 464 (M+H, 100%) |
| **36** | 6-[(2-aminoethyl)thio]-2-[[(2,3-difluorophenyl)methyl]thio]-4-[[(1R)-2-hydroxy-1-methylethyl]amino]-7(8H)-pteridinone | 453 (M+H, 100%) |

### Pharmacological Data

### Ligand Binding Assay

[¹²⁵I]IL-8 (human, recombinant) was purchased from Amersham, U.K. with a specific activity of 2,000Ci/mmol. All other chemicals were of analytical grade. High levels of hrCXCR2 were expressed in HEK 293 cells (human embryo kidney 293 cells ECACC No. 85120602) (Lee et al. (1992) J. Biol. Chem. 267 pp16283-16291). hrCXCR2 cDNA was amplified and cloned from human neutrophil mRNA. The DNA was cloned into PCRScript (Stratagene) and clones were identified using DNA. The coding sequence was sub-cloned into the eukaryotic expression vector RcCMV (Invitrogen). Plasmid DNA was prepared using Quiagen Megaprep 2500 and transfected into HEK 293 cells using Lipofectamine reagent (Gibco BRL). Cells of the highest expressing clone were harvested in phosphate-buffered saline containing 0.2%(w/v) ethylenediaminetetraacetic acid (EDTA) and centrifuged (200g, 5min.). The cell pellet was resuspended in ice cold homogenisation buffer [10mM HEPES (pH 7.4),1mM dithiothreitol, 1mM EDTA and a panel of protease inhibitors (1mM phenyl methyl sulphonyl fluoride, 2µg/ml soybean trypsin inhibitor, 3mM benzamidine, 0.5µg/ml leupeptin and 100µg/ml bacitracin)] and the cells left to swell for 10 minutes. The cell preparation was disrupted using a hand held glass mortar/PTFE pestle homogeniser and cell membranes harvested by centrifugation (45 minutes,100,000g, 4°C). The membrane preparation was stored at -70°C in homogenisation buffer supplemented with Tyrode's salt solution (137mM NaCl, 2.7mM KCl, 0.4mM NaH₂PO₄), 0.1%(w/v) gelatin and 10%(v/v) glycerol.

All assays were performed in a 96-well MultiScreen 0.45µm filtration plates (Millipore, U.K.). Each assay contained ~5OpM [¹²⁵I]IL-8 and membranes (equivalent to -200,000 cells) in assay buffer [Tyrode's salt solution supplemented with 10mM HEPES (pH 7.4), 1.8mM CaCl₂, 1mM MgCl₂, 0.125mg/ml bacitracin and 0.1%(w/v) gelatin]. In addition, a compound of formula (I) according to the Examples was pre-dissolved in DMSO and added to reach a final concentration of 1%(v/v) DMSO. The assay was initiated with the addition of membranes and after 1.5 hours at room temperature the membranes were harvested by filtration using a Millipore MultiScreen vacuum manifold and washed twice with assay buffer (without bacitracin). The backing plate was removed from the MultiScreen plate assembly, the filters dried at room temperature, punched out and then counted on a Cobra γ-counter.

The compounds of formula (I) according to the Examples were found to have IC₅₀ values of less than (<) 10µM.

### Intracellular Calcium Mobilisation Assay

Human neutrophils were prepared from EDTA-treated peripheral blood, as previously described (Baly et al. (1997) Methods in Enzymology 287 pp70-72), in storage buffer [Tyrode's salt solution (137mM NaCl, 2.7mM KCl, 0.4mM NaH₂PO₄) supplemented with 5.7mM glucose and 10mM HEPES (pH 7.4)].

The chemokine GROα (human, recombinant) was purchased from R&D Systems (Abingdon, U.K.). All other chemicals were of analytical grade. Changes in intracellular free calcium were measured fluorometrically by loading neutrophils with the calcium sensitive fluorescent dye, fluo-3, as described previously (Merritt et al. (1990) Biochem. J. 269, pp513-519). Cells were loaded for 1 hour at 37°C in loading buffer (storage buffer with 0.1%(w/v) gelatin) containing 5*µ*M fluo-3 AM ester, washed with loading buffer and then resuspended in Tyrode's salt solution supplemented with 5.7mM glucose, 0.1%(w/v) bovine serum albumin (BSA), 1.8mM CaCl₂ and 1mM MgCl₂. The cells were pipetted into black walled, clear bottom, 96 well micro plates (Costar, Boston, U.S.A.) and centrifuged (200g, 5 minutes, room temperature).

A compound of formula (I) according to the Examples was pre-dissolved in DMSO and added to a final concentration of 0.1%(v/v) DMSO. Assays were initiated by the addition of an A₅₀ concentration of GRO_{α} and the transient increase in fluo-3 fluorescence (λEx =490nm and λ_{Em} = 520nm) monitored using a FLIPR (Fluorometric Imaging Plate Reader, Molecular Devices, Sunnyvale, U.S.A.).

The compounds of formula (I) according to the Examples were tested and found to be antagonists of the CXCR2 receptor in human neutrophils.

## Claims

1. A compound of formula (I) or a pharmaceutically acceptable salt, solvate or *in vivo* hydrolysable ester thereof: 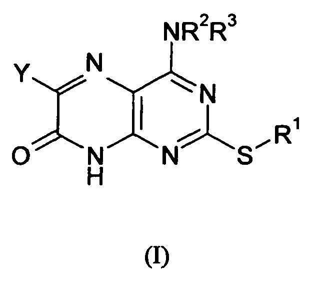 in which:
R¹ represents a C₃-C₇ carbocyclic, C₁-C₈ alkyl, C₂-C₆ alkenyl or C₂-C₆ alkynyl group, each of which may be optionally substituted by one or more substituent groups independently selected from halogen atoms, -OR⁴, -NR⁵R⁶, -CONR⁵R⁶, -COOR⁷, -NR⁸COR⁹, -SR¹⁰, -SO₂R¹⁰, -SO₂NR⁵R⁶, -NR⁸SO₂R⁹, an aryl or heteroaryl group, which last two may themselves be optionally substituted by one or more substituents independently selected from halogen atoms, cyano, nitro, -OR⁴, -NR⁵R⁶, -CONR⁵R⁶, -COOR⁷ -NR⁸COR⁹, -SR¹⁰, -SO₂R¹⁰, -SO₂NR⁵R⁶, -NR⁸SO₂R⁹, C₁-C₆ alkyl or trifluoromethyl groups;
R² and R³ each independently represent a hydrogen atom, or a C₃-C₇ carbocyclic, C₁-C₈ alkyl, C₂-C₆ alkenyl or C₂-C₆ alkynyl group, the latter four groups may be optionally substituted by one or more substituent groups independently selected from:
(a) halogen atoms, -OR⁴, -NR⁵R⁶ -CONR⁵R⁶, -COOR⁷, -NR⁸COR⁹, -SR¹⁰, -SO₂R¹⁰, -SO₂NR⁵R⁶, -NR⁸SO₂R⁹;
(b) a 3-8 membered ring optionally containing one or more atoms selected from O, S, NR⁸ and itself optionally substituted by C₁-C₃-alkyl or halogen; or
(c) an aryl group or heteroaryl group each of which may be optionally substituted by one or more substituents independently selected from halogen atoms, cyano, nitro, - OR⁴, - NR⁵R⁶, - CONR⁵R⁶, - NR⁸COR⁹, -SO₂NR⁵R⁶, -NR⁸SO₂R⁹, C₁-C₆ alkyl and trifluoromethyl groups;
R⁴ represents hydrogen or a C₁-C₆ alkyl group which may be optionally substituted by one or more substituent groups independently selected from halogen atoms, -OR¹¹, -NR⁵R⁶, or an aryl group or heteroaryl group either of which may be optionally substituted by one or more substituents independently selected from halogen atoms, cyano, nitro, -OR¹¹, -NR⁵R⁶, - CONR⁵R⁶, -NR⁸COR⁹, -SO₂NR⁵R⁶, -NR⁸SO₂R⁹, C₁-C₆ alkyl and trifluoromethyl groups; or R⁴ represents a halogen atom, -OR¹¹, -NR⁵R⁶, or an aryl group or heteroaryl group either of which may be optionally substituted by one or more substituents independently selected from halogen atoms, cyano, nitro, -OR¹¹, -NR⁵R⁶, -CONR⁵R⁶, -NR⁸COR⁹, -SO₂NR⁵R⁶, - NR⁸SO₂R⁹, C₁-C₆ alkyl and trifluoromethyl groups;
R⁵ and R⁶ independently represent a hydrogen atom or a C₁-C₆ alkyl or phenyl group or heteroaryl group the latter three of which may be optionally substituted by one or more substituent groups independently selected from halogen atoms, phenyl, -OR¹⁴ and -NR¹⁵R¹⁶, -CONR¹⁵R¹⁶, -NR¹⁵COR¹⁶, -SONR¹⁵R¹⁶, NR¹⁵SO₂R¹⁶
or
R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from phenyl, -OR¹⁴, -COOR¹⁴, -NR¹⁵R¹⁶ , -CONR¹⁵R¹⁶, -NR¹⁵COR¹⁶, -SONR¹⁵R¹⁶, NR¹⁵SO₂R¹⁶ or C₁-C₆ alkyl, itself optionally substituted by one or more substituents independently selected from halogen atoms and - NR¹⁵R¹⁶ and -OR¹⁷ groups;R¹⁰ represents a C₁-C₆-alkyl or a phenyl group, either of which may be optionally substituted by one or more substituent groups independently selected from halogen atoms, phenyl, -OR¹⁷ and -NR¹⁵R¹⁶,
Y is NR²⁰R²¹, OR⁴, SR⁴, a heteroaryl group or NR⁵R⁶ where R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from phenyl, -OR¹⁴, -COOR¹⁴, -NR¹⁶R¹⁶, -CONR¹⁵R¹⁶, -NR¹⁵COR¹⁶, -SONR¹⁵R¹⁶, NR¹⁵SO₂R¹⁶ or C₁-C₆ alkyl, itself optionally substituted by one or more substituents independently selected from halogen atoms and -NR¹⁵R¹⁶ and -OR¹⁷ groups; each of R⁷, R⁸, R⁹, R¹¹, R¹⁴ R¹⁵, R¹⁶ and R¹⁷ independently represents a hydrogen atom or a C₁-C₆, alkyl, or a phenyl group;
and R²⁰ and R²¹ are defined as for R² and R³.

2. A compound according to claim 1, wherein R¹ represents an optionally substituted benzyl group.

3. A compound according to claim 2, wherein R¹ represents benzyl substituted by two halogen atoms.

4. A compound according to any one of claims 1 to 3, wherein one of R² and R³ is hydrogen and the other is C₃-C₄ alkyl substituted by one or more hydroxy groups.

5. A compound according to any one of claims 1 to 4 wherein one of R² and R³ is hydrogen and the other is CH(CH₃)CH₂OH, CH(Et)CH₂OH, C(CH₃)₂CH₂OH or CH(CH₂OH)₂.

6. A compound according to any one of claims 1 to 4 wherein one of R² and R³ is hydrogen and the other is CH(CH₃)CH₂OH.

7. A compound according to claim 6 in the form of the (R) isomer.

8. A compound according to any one of claims 1 to 7 wherein Y is -NR²⁰R²¹, -OR⁴, -SR⁴, a heteroaryl group or -NR⁵R⁶ where R⁵ and R⁶ together with the nitrogen atom to which they are attached form a 4- to 7-membered saturated heterocyclic ring system optionally containing a further heteroatom selected from oxygen and nitrogen atoms, which ring system may be optionally substituted by one or more substituent groups independently selected from -OH, -NH₂ or C₁-C₄ alkyl.

9. A compound according to claim 1 selected from:
2-[[(2,3-difluorophenyl)methyl]thio]-6-[(2-hydroxyethyl)amino]-4-[[(1*R*)-2-hydroxy-1-methylethyl]amino]-7(8*H*)-pteridinone;
2-[[(2,3-difluorophenyl)methyl]thio]-4-[[(1*R*)-2-hydroxy-1-methylethyl]amino]-6-[(phenylmethyl)amino]-7(8*H*-pteridinone;
2-[[(2,3-Difluorophenyl)methyl]thio-4-[[(1R)-2-hydroxy-1-methylethyl]amino]-6,7-pteridinedione;
6-amino-2-[[(2,3-difluorophenyl)methyl]thio]-4-[[(1*R*)-2-hydroxy-1-methylethyl]amino]-7(8*H*)-pteridinone;
2-[[2,3-difluorophenyl)methyl)thio]-4-[[(1R)-2-hydroxy-1-methylethyl]amino]-6-(1H-imidazol-1-yl)-7(8H)-pteridinone;
2-[[2,3-difluorophenyl)methyl)thio]-4-[[(1R)-2-hydroxy-1-methylethyl]amino]-6-[(1-methyl-1H-imidazol-2-yl)thio]-7(8H)-pteridinone;
2-[[2,3-difluorophenyl)methyl)thio]-4-[[(1R)-2-hydroxy-1-methylethyl] amino]-6-methoxy-7(8H)-pteridinone;
2-[[(2,3-difluorophenyl)methyl]thio]-4-[[(1R)-2-hydroxy-1-methylethyl]amino]-6-[(3-pyridinylmethyl)amino]-7(8H)-pteridinone;
2-[[(2,3-difluorophenyl)methyl]thio]-4-[[(1R)-2-hydroxy-1-methylethyl]amino]-6-[[(5-methyl-2-furanyl)methyl]amino]-7(8H)-pteridinone;
2-[[(2,3-difluorophenyl)methyl]thio]-6-[(3R,5S)-3,5-dimethyl-1-piperazinyl]-4-[[(1R)-2-hydroxy-1-methylethyl] amino]-7(8H)-pteridinone;
2-[[(2,3-difluorophenyl)methyl]thio]-4-[[(1R)-2-hydroxy-1-methylethyl]amino]-6-[methyl[(3-methyl-5-isoxazolyl)methyl] amino]-7(8H)-pteridinone;
2-[[(2,3-difluorophenyl)methyl]thio]-4-[[(1R)-2-hydroxy-1-methylethyl]amino]-6-[[2-(2-pyrimidinylamino)ethyl]amino]-7(8H)-pteridinone;
2-[[(2,3-difluorophenyl)methyl]thio]-4-[[(1R)-2-hydroxy-1-methylethyl]amino]-6-(4-morpholinyl)-7(8H)-pteridinone;
2-[[(2,3-difluorophenyl)methyl]thio]-4-[[(1R)-2-hydroxy-1-methylethyl]amino]-6-[[2-(4-morpholinyl)ethyl]amino]-7(8H)-pteridinone;
2-[[(2,3-difluorophenyl)methyl]thio]-4-[[(1R)-2-hydroxy-1-methylethyl]amino]-6-[(2-methoxyethyl)amino]-7(8H)-pteridinone;
2-[[(2,3-difluorophenyl)methyl]thio]-6-[(2-furanylmethyl)amino]-4-[[(1R)-2-hydroxy-1-methylethyl]amino]-7(8H)-ptendinone;
6-(1-azetidinyl)-2-[[(2,3-difluorophenyl)methyl]thio]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[[(5-methylpyrazinyl)methyl]amino]-7(8H)-pteridinone;
2-[[(2,3-difluorophenyl)methyl]thio]-6-[[2-(2-furanyl)ethyl]amino]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[[3-(4-morpholinyl)propyl]amino]-7(8H)-pteridinone;
2-[[(2,3-difluorophenyl)methyl]thio]-4-[[(1R)-2-hydroxy-1-methylethyl]amino]-6-[[(3-methyl-5-isoxazolyl)methyl]amino]-7(8H)-pteridinone;
2-[[(2,3-difluorophenyl)methyl]thio]-4-[[(1R)-2-hydroxy-1-methylethyl]amino]-6-[(3S)-3-hydroxy-1-pyrrolidinyl]-7(8H)-pteridinone;
2-[[(2,3-difluorophenyl)methyl]thio]-6-[(2-furanylmethyl)thio]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[(2-hydroxypropyl)amino]-7(8H)-pteridinone;
2-[[(2,3-difluorophenyl)methyl]thio]-6-[[2-(dimethylamino)ethyl]thio]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[[(2S)-2-hydroxypropyl]amino]-7(8H)-pteridinone;
2-[[(2,3-difluorophenyl)methyl]thio]-4-[[(1R)-2-hydroxy-1-methylethyl]amino]-6-[(3-hydroxypropyl)amino]-7(8H)-pteridinone;
2-[[(2,3-difluorophenyl)methyl]thio]-6-[(2-hydroxyethyl)methylamino]-4-[[(1R)-2-hydroxy-1-methylethyl]amino]-7(8H)-pteridinone;
2-[[(2,3-difluorophenyl)methyl]thio]-4-[[(1R)-2-hydroxy-1-methylethyl]amino]-6-[(5-hydroxy-4-methyl-4H-1,2,4-triazol-3-yl)thio]-7(8H)-pteridinone;
2-[[(2,3-difluorophenyl)methyl]thio]-6-[(4-hydroxycyclohexyl)amino]-4-[[(1R)-2-hydroxy-1-methylethyl]amino-7(8H)-pteridinone;
2-[[(2,3-difluorophenyl)methyl]thio]-4-[[(1R)-2-hydroxy-1-methylethyl]amino]-6-(1,3,4-thiadiazol-2-ylthio)-7(8H)-pteridinone;
2-[[(2,3-difluorophenyl)methyl]thio]-6-[[(1S,4R)-4-hydroxy-2-cyclopenten-1-yl]amino]-4-[[(1R)-2-hydroxy-1-methyl]ethyl] amino]-7(8H-pteridinone;
2-[[(2,3-difluorophenyl)methyl]thio]-4-([(1R)-2-hydroxy-1-methylethy1]amino]-6-[(3R)-3-hydroxy-1-xy-1-pyrrolidinyl]-7(8H)-pteridinone;
2-[[(2,3-difluorophenyl)methyl]thio]-6-(3-hydroxy-3-methy]-1-azetidmyl)-4-[[(1R)-2-hydroxy-1-methylethyl]amino]-7(8H)-pteridinone;
6-[(3S)-3-amino-1-pyrrolidinyl]-2-[[(2,3-difluorophenyl)methyl]thio]-4-[[(1R)-2-hydroxy-1-methylethyl]amino]-7(8H)-pteridinone; and
6-[(2-aminoethyl)thio]-2-[[(2,3-difluorophenyl)methyl]thio]-4-[[(1R)-2-hydroxy-1-methylethyl]amino]-7(8H)-pteridinone.

10. A process for the preparation of:
(a) a compound of formula (1) as defined in claim 1 where Y is NR²⁰R²¹ which comprises treatment of a compound of formula (ITA): 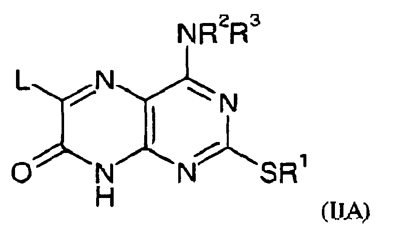 where R¹, R² and R³ are as defined in formula (I) or are protected derivatives thereof and L is a leaving group selected from hydroxy, bromo or chloro, with an amine HNR²⁰R²¹, or
(b) a compound of formula (I) as defined in claim 1 where Y is OR⁴ which comprises treatment of a compound of formula (IIA) where R¹, R² and R³ are as defined in formula (I) or are protected derivatives thereof and L is a leaving group selected from hydroxy, bromo or chloro with an alcohol R⁴OH, or
(c) a compound of formula (I) as defined in claim 1 where Y is SR⁴ which comprises treatment of a compound of formula (IIA) where R¹, R² and R³ are as defined in formula (I) or are protected derivatives thereof and L is a leaving group selected from hydroxy, bromo or chloro with a thiol R⁴SH, or
(d) a compound of formula (I) where Y is NR⁵R⁶ which comprises treatment of a compound of formula (IIA) where R¹, R² and R³ are as defined in formula (I) or are protected derivatives thereof and L is a leaving group selected from hydroxy, bromo or chloro with an amine
H NR⁵R⁶, or
(e) a compound of formula (I) where Y is a heteroaryl group which comprises treatment of a compound of formula (IIA) where R¹, R² and R³ are as defined in formula (I) or are protected derivatives thereof and L is a leaving group selected from hydroxy, bromo or chloro with a heteroarene, or
(f) a compound of formula (1) as defined in claim 1 where Y is OH which comprises treatment of a compound of formula (IIB): 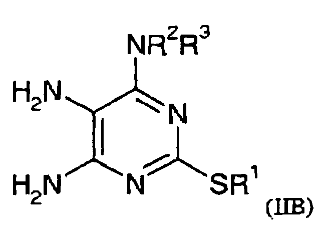 where R¹, R² and R³ are as defined in formula (I) or are protected derivatives thereof with diethyl oxalate, or
(g) a compound of formula (I) as defined in claim 1 where Y is NH₂ which comprises treatment of a compound of formula (IIB) where R¹, R² and R³ are as defined in formula (I) or are protected derivatives thereof with iminomethoxy-acetic acid, methyl ester hydrochloride, and optionally thereafter process (a), (b), (c), (d) or (e) and in any order:
• removing any protecting groups
• forming a pharmaceutically acceptable salt, solvate or *in vivo* hydrolysable ester.

11. An intermediate compound of formula (IIA) as defined in claim 10.

12. A pharmaceutical composition comprising a compound of formula (I), or a pharmaceutically acceptable salt, solvate or *in vivo* hydrolysable ester thereof, as claimed in any one of claims 1 to 9 in association with a pharmaceutically acceptable adjuvant, diluent or carrier.

13. A process for the preparation of a pharmaceutical composition as claimed in claim 12 which comprises mixing a compound of formula (I), or a pharmaceutically acceptable salt, solvate or *in vivo* hydrolysable ester thereof, as claimed in any one of claims 1 to 9 with a pharmaceutically acceptable adjuvant, diluent or carrier.

14. A compound of formula (I), or a pharmaceutically-acceptable salt, solvate or *in vivo* hydrolysable ester thereof, as claimed in any one of claims 1 to 9 for use in therapy.

15. Use of a compound of formula (I), or a pharmaceutically acceptable salt, solvate or *in vivo* hydrolysable ester thereof, as claimed in any one of claims 1 to 9 in the manufacture of a medicament for use in therapy.

## Patentansprüche

1. Verbindung der Formel (I) oder ein pharmazeutisch annehmbares Salz oder Solvat oder ein pharmazeutisch annehmbarer in vivo hydrolysierbarer Ester davon: 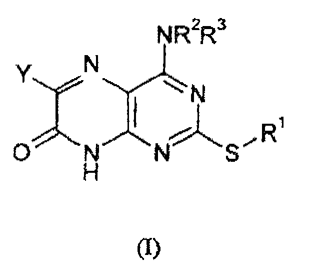 worin
R¹ für eine C₃-C₇-carbocyclische Gruppe, C₁-C₈-Alkyl-, C₂-C₆-Alkenyl- oder C₂-C₆-Alkinylgruppe steht, wobei jede dieser Gruppen gegebenenfalls durch eine oder mehrere unabhängig voneinander unter Halogenatomen, -OR⁴, -NR⁵R⁶, - CONR⁵R⁶, - COOR⁷, -NR⁸COR⁹-SR¹⁰, -SO₂R¹⁰, -SO₂NR⁵R⁶, -NR⁸SO₂R⁹, oder einer gegebenenfalls selbst durch eine oder mehrere unabhängig voneinander unter Halogenatomen, Cyano, Nitro, -OR⁴⁻, -NR⁵R⁶-, -CONR⁵R⁶-, -COOR⁷-NR⁸COR⁹-, -SR¹⁰-, -SO₂R¹⁰-, -SO₂NR⁵R⁶-, -NR⁸SO₂R⁹-, C₁-C₆-Alkyl- und Trifluormethylgruppen ausgewählte Substituenten substituierten Aryl- oder Heteroarylgruppe ausgewählte Substituentengruppen substituiert ist;
R² und R³ jeweils unabhängig voneinander für ein Wasserstoffatom oder eine C₃-C₇-carbocyclische Gruppe, C₁-C₈-Alkyl-, C₂-C₆-Alkenyl- oder C₂-C₆-Alkinylgruppe stehen, wobei die letzten vier Gruppen gegebenenfalls durch eine oder mehrere unabhängig voneinander unter:
(a) Halogenatomen, -OR⁴, -NR⁵R⁶, - CONR⁵R⁶ , -COOR⁷, -NR⁸COR⁹, -SR¹⁰, -SO₂R¹⁰, -SO₂NR⁵R⁶, -NR⁸SO₂R⁹;
(b) einem 3-8-gliedrigen Ring, der gegebenenfalls ein oder mehrere unter O, S, NR⁸ ausgewählte Atome enthält und gegebenenfalls selbst durch C₁₋₃-Alkyl oder Halogen substituiert ist; oder
(c) einer gegebenenfalls durch einen oder mehrere unabhängig voneinander unter Halogenatomen, Cyano, Nitro, -OR⁴-, -NR⁵R⁶-, -CONR⁵R⁶-, -NR⁸COR⁹-, -SO₂NR⁵R⁶-, -NR⁸SO₂R⁹-, C₁-C₆-Alkyl- und Trifluormethylgruppen ausgewählte Substituenten substituierten Aryl- oder Heteroarylgruppe
ausgewählte Substituentengruppen substituiert sein können;
R⁴ für Wasserstoff oder eine gegebenenfalls durch eine oder mehrere unabhängig voneinander unter Halogenatomen, -OR¹¹, -NR⁵R⁶ oder einer gegebenenfalls durch eine oder mehrere unabhängig voneinander unter Halogenatomen, Cyano, Nitro, -OR¹¹-, -NR⁵R⁶- , -CONR⁵R⁶-, -NR⁸COR⁹- , -SO₂NR⁵R⁶- , -NR⁸SO₂R⁹-, C₁-C₆-Alkyl- und Trifluormethylgruppen ausgewählte Substituenten substituierten Aryl- oder Heteroarylgruppe ausgewählte Substituentengruppen substituierte C₁-C₆-Alkylgruppe steht; oder R⁴ für ein Halogenatom, - OR¹¹, -NR⁵R⁶ oder eine gegebenenfalls durch einen oder mehrere unabhängig voneinander unter Halogenatomen, Cyano, Nitro, -OR¹¹-, -NR⁵R⁶-, CONR⁵R⁶-, -NR⁸COR⁹-, -SO₂NR⁵R⁶-, -NR⁸SO₂R⁹-, C₁-C₆-Alkyl- und Trifluormethylgruppen ausgewählte Substituenten substituierte Aryl- oder Heteroarylgruppe steht;
R⁵ und R⁶ unabhängig voneinander für ein Wasserstoffatom oder eine C₁-C₆-Alkyl-, Phenyl- oder Heteroarylgruppe stehen, wobei die letzten drei Gruppen gegebenenfalls durch eine oder mehrere unabhängig voneinander unter Halogenatomen, Phenyl, -OR¹⁴ und -NR¹⁵R¹⁶, -CONR¹⁵R¹⁶, -NR¹⁵COR¹⁶, -SONR¹⁵R¹⁶ und NR¹⁵SO₂R¹⁶ ausgewählte Substituentengruppen substituiert sind, oder
R⁵ und R⁶ gemeinsam mit dem Stickstoffatom, an das sie gebunden sind, ein 4- bis 7- gliedriges gesättigtes heterocyclisches Ringsystem bilden, welches gegebenenfalls ein weiteres unter Sauerstoff- und Stickstoffatomen ausgewähltes Heteroatom enthält und gegebenenfalls durch eine oder mehrere unabhängig voneinander unter Phenyl, - OR¹⁴, -COOR¹⁴, -NR¹⁵R¹⁶, -CONR¹⁵R¹⁶, -NR¹⁵COR¹⁶, -SONR¹⁵R¹⁶, NR¹⁵SO₂R¹⁶ oder gegebenenfalls selbst durch einen oder mehrere unabhängig voneinander unter Halogenatomen und -NR¹⁵R¹⁶- und -OR¹⁷-Gruppen ausgewählte Substituenten substituiertes C₁-C₆-Alkyl ausgewählte Substituentengruppen substituiert ist; R¹⁰ für eine C₁-C₆-Alkyl- oder Phenylgruppe steht, die jeweils gegebenenfalls durch eine oder mehrere unabhänging voneinander unter Halogenatomen, Phenyl, -OR¹⁷ und -NR¹⁵R¹⁶ ausgewählte Substituentengruppen substituiert ist;
Y für NR²⁰R²¹, OR⁴, SR⁴, eine Heteroarylgruppe oder NR⁵R⁶ steht, wobei R⁵ und R⁶ gemeinsam mit dem Stickstoffatom, an das sie gebunden sind, ein 4-bis 7-gliedriges gesättigtes heterocyclisches Ringsystem bilden, welches gegebenenfalls ein weiteres unter Sauerstoff- und Stickstoffatomen ausgewähltes Heteroatom enthält und gegebenenfalls durch eine oder mehrere unabhängig voneinander unter Phenyl, -OR¹⁴, -COOR¹⁴, -NR¹⁵R¹⁶, -CONR¹⁵R¹⁶, - NR¹⁵COR¹⁶, - SONR¹⁵R¹⁶, NR¹⁵SO₂R¹⁶ oder gegebenenfalls selbst durch einen oder mehrere unabhängig voneinander unter Halogenatomen und -NR¹⁵R¹⁶- und -OR¹⁷-Gruppen ausgewählte Substituenten substituiertes C₁-C₆-Alkyl ausgewählte Substituentengruppen substituiert ist;
R⁷, R⁸, R^{9,} R¹¹, R¹⁴, R¹⁵ R¹⁶ und R¹⁷ jeweils unabhängig voneinander für ein Wasserstoffatom oder eine C₁-C₆-Alkyl- oder Phenylgruppe stehen und R²⁰ und R²¹ die gleiche Bedeutung wie R² und R³ besitzen.

2. Verbindung nach Anspruch 1, worin R¹ für eine gegebenenfalls substituierte Benzylgruppe steht.

3. Verbindung nach Anspruch 2, worin R¹ für durch zwei Halogenatome substituiertes Benzyl steht.

4. Verbindung nach einem der Ansprüche 1 bis 3, worin eine der Gruppen R² und R³ für Wasserstoff steht und die andere für durch eine oder mehrere Hydroxygruppen substituiertes C₃-C₄-Alkyl steht.

5. Verbindung nach einem der Ansprüche 1 bis 4, worin eine der Gruppen R² und R³ für Wasserstoff steht und die andere für CH(CH₃)CH₂OH, CH (Et) CH₂OH, C(CH₃)₂CH₂OH oder CH(CH₂OH)₂ steht.

6. Verbindung nach einem der Ansprüche 1 bis 4, worin eine der Gruppen R² und R³ für Wasserstoff steht und die andere für CH(CH₃)CH₂OH steht.

7. Verbindung nach Anspruch 6 in Form des (R)-Isomers.

8. Verbindung nach einem der Ansprüche 1 bis 7, worin Y für -NR²⁰R²¹, -OR⁴, -SR⁴, eine Heteroarylgruppe oder -NR⁵R⁶ steht, wobei R⁵ und R⁶ gemeinsam mit dem Stickstoffatom, an das sie gebunden sind, ein 4-bis 7-gliedriges gesättigtes heterocyclisches Ringsystem bilden, welches gegebenenfalls ein weiteres unter Sauerstoff- und Stickstoffatomen ausgewähltes Heteroatom enthält und gegebenenfalls durch eine oder mehrere unabhängig voneinander unter -OH, -NH₂ oder C₁-C₄-Alkyl ausgewählte Substituentengruppen substituiert ist.

9. Verbindung nach Anspruch 1, ausgewählt unter:
2-[[(2,3-Difluorphenyl)methyl]thio]-6-[(2-hydroxyethyl)amino]-4-[[(1R)-2-hydroxy-1-methylethyl]-amino]-7(8H)-pteridinon ;
2-[[(2,3-Difluorphenyl)methyl]thio]-4-[[(1R)-2-hydroxy-1-methylethyl]amino]-6-[(phenylmethyl)-amino]-7(8H)-pteridinon;
2-[[(2,3-Difluorphenyl)methyl]thio]-4-[[(1R)-2-hydroxy-1-methylethyl]amino]-6,7-pteridindion ;
6-Amino-2-[[(2,3-difluorphenyl)methyl]thio]-4-[[(1R)-2-hydroxy-1-methylethyl]amino]-7(8H)-pteridinon;
2-[[(2,3-Difluorphenyl)methyl]thio]-4-[[(1R)-2-hydroxy-1-methylethyl]amino]-6-(1H-imidazal-1-yl)-7(8H)-pteridinon;
2-[[(2,3-Difluorphenyl)methyl]thio]-4-[[(1R)-2-hydroxy-1-methylethyl]amino]-6-[(1-methyl-1H-imidazol-2-yl)thio]-7(8H)-pteridinon;
2-[[(2,3-Difluorphenyl)methyl]thio]-4-[[(1R)-2-hydroxy-1-methylethyl]amino]-6-methaxy-7(8H)-pteridinon;
2-[[(2,3-Difluorphenyl)methyl]thio]-4-[[(1R)-2-hydroxy-1-methylethyl]amino]-6-[(3-pyridinylmethyl)amino]-7(8H)-pteridinon;
2-[[(2,3-Difluorphenyl)methyl]thio]-4-[[(1R)-2-hydroxy-1-methylethyl]amino]-6-[[(5-methyl-2-furanyl)methyl]amino]-7(8H)-pteridinon;
2-[[(2,3-Difluorphenyl)methyl]thio]-6-[(3R,5S)-3,5-dimethyl-1-piperazinyl]-4-[[(1R)-2-hydroxy-1-methylethyl]amino]-7(8H)-pteridinon;
2-[[(2,3-Difluorphenyl)methyl]thio]-4-[[(1R)-2-hydroxy-1-methylethyl]amino]-6-[methyl[(3-methyl-5-isoxazolyl)methyl]amino]-7(8H)-pteridinon;
2-[[(2,3-Difluorphenyl)methyl]thio]-4-[[(1R)-2-hydroxy-1-methylethyl] amino] -6-[[2- (2-pyrimidinylamino)ethyl]amino]-7(8H)-pteridinon;
2-[[(2,3-Difluorphenyl)methyl]thio]-4-[[(1R)-2-hydroxy-1-methylethyl]amino]-6-(4-morpholinyl)-7(8H)-pteridinon;
2- [[(2,3-Difluorphenyl)methyl]thio] -4- [[(1R)-2-hydroxy-1-methylethyl] amino] -6- [[2- (4-morpholinyl)ethyl]amino]-7(8H)-pteridinon;
2-[[(2,3-Difluorphenyl)methyl]thiol-4-[[(1R)-2-hydroxy-1-methylethyl]amino]-6-[(2-methaxyethyl)-amino]-7(8H)-pteridinon;
2-[[(2,3-Difluorphenyl)methyl]thio]-6-[(2-furanylmethyl)amino]-4-[[(1R)-2-hydroxy-1-methylethyl]-amino]-7(8H)-pteridinon;
6-(1-Azetidinyl)-2-[[(2,3-difluorphenyl)methyl]-thio]-4-[[(1R)-2-hydroxy-1-methylethyl]amino]-7(8H)-pteridinon;
2-[[(2,3-Difluorphenyl)methyl]thiol-4-[[(1R)-2-hydroxy-1-methylethyl]amino]-6-[[(5-methyl-pyrazinyl)methyl]amino]-7(8H)-pteridinon;
2-[[(2,3-Difluorphenyl)methyl]thio]-6-[[2-(2-furanyl)ethyl]amino]-4-[[(1R)-2-hydroxy-1-methylethyl]amino]-7(8H)-pteridinon;
2-[[(2,3-Difluorphenyl)methyl]thio]-4-[[(1R)-2-hydroxy-1-methylethyl]amino]-6-[[3-(4-morpholinyl)propyl]amino]-7(8H)-pteridinon;
2-[[(2,3-Difluorphenyl)methyl]thio]-4-[[(1R)-2-hydroxy-1-methylethyl]amino]-6-[[(3-methyl-5-isoxazolyl)methyl]amino]-7(8H)-pteridinon;
2-[[(2,3-Difluorphenyl)methyl]thio]-4-[[(1R)-2-hydroxy-1-methylethyl]amino]-6-[(3S)-3-hydroxy-1-pyrrolidinyl]-7(8H)-pteridinon;
2-[[(2,3-Difluorphenyl)methyl]thio]-6-[(2-furanylmethyl)thiol-4-[[(1R)-2-hydroxy-1-methylethyl]-amino]-7(8H)-pteridinon;
2-[[(2,3-Difluorphenyl)methyl]thio]-4-[[(1R)-2-hydroxy-1-methylethyl]amino]-6-[(2-hydroxypropyl)-amino]-7(8H)-pteridinon;
2-[[(2,3-Difluorphenyl)methyl] thio]-6-[[(2-(dimethylamino)ethyl]thio]-4-[[(1R)-2-hydroxy-1-methylethyl]amino]-7(8H)-pteridinon;
2-[[(2,3-Difluorphenyl)methyl]thio]-4-[[(1R)-2-hydroxy-1-methylethyl]amino]-6-[[(2S)-2-hydroxypropyl]amino]-7(8H)-pteridinon;
2-[[(2,3-Difluorphenyl)methyl]thio]-4-[[(1R)-2-hydroxy-1-methylethyl]amino]-6-[(3-hydroxypropyl)-amino]-7(8H)-pteridinon;
2-[[(2,3-Difluorphenyl)methyllthiol-6-[(2-hydroxyethyl)methylamino]-4-[[(1R)-2-hydroxy-1-methylethyl]amino]-7(8H)-pteridinon;
2-[[(2,3-Difluorphenyl)methyl]thio]-4-[[(1R)-2-hydroxy-1-methylethyl]amino]-6-[(5-hydroxy-4-methyl-4H-1,2,4-triazol-3-yl)thio]-7(8H)-pteridinon;
2-[[(2,3-Difluorphenyl)methyl]thio]-6-[(4-hydroxy-cyclohexyl)amino]-4-[[(1R)-2-hydroxy-1-methylethyl]amino]-7(8H)-pteridinon;
2-[[(2,3-Difluorphenyl)methyl]thio]-4-[[(1R)-2-hydroxy-1-methylethyl]amino]-6-(1,3,4-thiadiazol-2-ylthio)-7(8H)-pteridinon;
2-[[(2,3-Difluorphenyl)methyl]thio]-6-[[(1S,4R)-4-hydroxy-2-cyclopenten-1-y1]amino]-4-[[(1R)-2-hydroxy-1-methylethyl]amino]-7(8H)-pteridinon;
2-[[(2,3-Difluorphenyl)methyl]thio]-4-[[(1R)-2-hydroxy-1-methylethyl]amino]-6-[(3R-3-hydroxy-1-pyrrolidinyl]-7(8H)-pteridinon;
2-[[(2,3-Difluorphenyl)methyl]thio]-6-(3-hydroxy-3-methyl-1-azetidinyl)-4-[[(1R)-2-hydroxy-1-methylethyl]amino]-7(8H)-pteridinon;
6-[(3S)-3-Amino-1-pyrrolidinyl]-2-[[(2,3-difluorphenyl)methyl]thio]-4-[[(1R)-2-hydroxy-1-methylethyl] amino]-7(8H)-pteridinon und
6-[(2-Aminoethyl)thio]-2-[[(2,3-difluorphenyl)-methyl]thio]-4-[[(1R)-2-hydroxy-1-methylethyl]-amino]-7(8H)-pteridinon.

10. Verfahren zur Herstellung
(a) einer Verbindung der Formel (I) gemäß Anspruch 1, worin Y für NR²⁰R²¹ steht, bei dem man eine Verbindung der Formel (IIA): 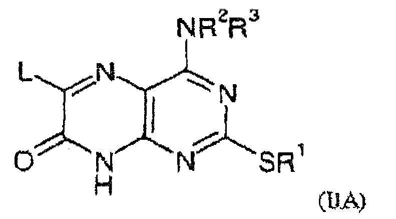 worin R¹, R² und R³ die unter Formel (I) angegebene Bedeutung besitzen oder für geschützte Derivate davon stehen und L für eine unter Hydroxy, Brom oder Chlor ausgewählte Abgangsgruppe steht, mit einem Amin HNR²⁰R²¹ behandelt, oder
(b) einer Verbindung der Formel (I) gemäß Anspruch 1, worin Y für OR⁴ steht, bei dem man eine Verbindung der Formel (IIA), worin R¹, R² und R³ die unter Formel (I) angegebene Bedeutung besitzen oder für geschützte Derivate davon stehen und L für eine unter Hydroxy, Brom oder Chlor ausgewählte Abgangsgruppe steht, mit einem Alkohol R⁴OH behandelt, oder
(c) einer Verbindung der Formel (I) gemäß Anspruch 1, worin Y für SR⁴ steht, bei dem man eine Verbindung der Formel (IIA), worin R¹, R² und R³ die unter Formel (I) angegebene Bedeutung besitzen oder für geschützte Derivate davon stehen und L für eine unter Hydroxy, Brom oder Chlor ausgewählte Abgangsgruppe steht, mit einem Thiol R⁴SH behandelt, oder
(d) einer Verbindung der Formel (I), worin Y für NR⁵R⁶ steht, bei dem man eine Verbindung der Formel (IIA), worin R¹, R² und R³ die unter Formel (I) angegebene Bedeutung besitzen oder für geschützte Derivate davon stehen und L für eine unter Hydroxy, Brom oder Chlor ausgewählte Abgangsgruppe steht, mit einem Amin HNR⁵R⁶ behandelt, oder
(e) einer Verbindung der Formel (I), worin Y für eine Heteroarylgruppe steht, bei dem man eine Verbindung der Formel (IIA), worin R¹, R² und R³ die unter Formel (I) angegebene Bedeutung besitzen oder für geschützte Derivate davon stehen und L für eine unter Hydroxy, Brom oder Chlor ausgewählte Abgangsgruppe steht, mit einem Heteroaren behandelt, oder
(f) einer Verbindung der Formel (I) gemäß Anspruch 1, worin Y für OH steht, bei dem man eine Verbindung der Formel (IIB): 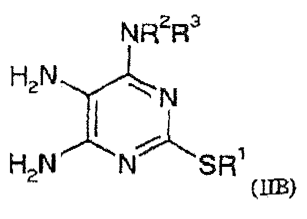 worin R¹, R² und R³ die unter Formel (I) angegebene Bedeutung besitzen oder für geschützte Derivate davon stehen, mit Oxalsäurediethylester behandelt, oder
(g) einer Verbindung der Formel (I) gemäß Anspruch 1, worin Y für NH₂ steht, bei dem man eine Verbindung der Formel (IIB), worin R¹, R² und R³ die unter Formel (I) angegebene Bedeutung besitzen oder für geschützte Derivate davon stehen, mit Iminomethoxyessigsäuremethylester-hydrochlorid behandelt,
und gegebenenfalls nach Verfahren (a), (b), (c), (d) oder (e) und in beliebiger Reihenfolge:
• Schutzgruppen abspaltet
• ein pharmazeutisch annehmbares Salz oder Solvat oder einen pharmazeutisch annehmbaren in vivo hydrolysierbaren Ester bildet.

11. Zwischenverbindung der Formel (IIA) gemäß Anspruch 10.

12. Pharmazeutische Zusammensetzung, die eine Verbindung der Formel (I) oder ein pharmazeutisch annehmbares Salz oder Solvat oder einen pharmazeutisch annehmbaren in vivo hydrolysierbaren Ester davon nach einem der Ansprüche 1 bis 9 zusammen mit einem pharmazeutisch annehmbaren Hilfsstoff, Verdünnungsmittel oder Träger enthält.

13. Verfahren zur Herstellung einer pharmazeutischen Zusammensetzung nach Anspruch 12, bei dem man eine Verbindung der Formel (I) oder ein pharmazeutisch annehmbares Salz oder Solvat oder einen pharmazeutisch annehmbaren in vivo hydrolysierbaren Ester davon nach einem der Ansprüche 1 bis 9 mit einem pharmazeutisch annehmbaren Hilfsstoff, Verdünnungsmittel oder Träger vermischt.

14. Verbindung der Formel (I) oder ein pharmazeutisch annehmbares Salz oder Solvat oder ein pharmazeutisch annehmbarer in vivo hydrolysierbarer Ester davon nach einem der Ansprüche 1 bis 9 zur Verwendung bei der Therapie.

15. Verwendung einer Verbindung der Formel (I) oder eines pharmazeutisch annehmbaren Salzes, Solvats oder in vivo hydrolysierbaren Esters davon nach einem der Ansprüche 1 bis 9 bei der Herstellung eines Arzneimittels zur Verwendung bei der Therapie.

## Revendications

1. Composé de formule (I) ou sel pharmaceutiquement acceptable, solvate ou ester hydrolysable in *vivo* de celui-ci: 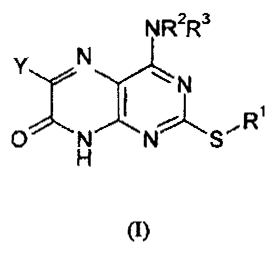 dans laquelle :
R¹ représente un groupement C₃₋₇ carbocyclique, C₁₋₈ alkyle, C₂₋₆ alcényle ou C₂₋₆ alcynyle, dont chacun peut être éventuellement substitué par un ou plusieurs groupements substituants choisis indépendamment parmi des atomes d'halogène ou un groupement -OR⁴, -NR⁵R⁶, -CONR⁵R⁶, -COOR⁷, -NR⁸COR⁹, -SR¹⁰, -SO₂R¹⁰, -SO₂NR⁵R⁶, -NR⁸SO₂R⁹, aryle ou hétéroaryle, les deux derniers pouvant eux-mêmes être éventuellement substitués par un ou plusieurs substituants choisis indépendamment parmi des atomes d'halogène ou des groupements cyano, nitro, -OR⁴, -NR⁵R⁶, -CONR⁵R⁶, -COOR⁷, -NR⁸COR⁹, -SR¹⁰, -SO₂R¹⁰ , -SO₂NR⁵R⁶, -NR⁸SO₂R⁹, C₁₋₆ alkyle ou trifluorométhyle ;
R² et R³ représentent chacun indépendamment un atome d'hydrogène ou un groupement C₃₋₇ carbocyclique, C₁₋₈ alkyle, C₂₋₆ alcényle ou C₂₋₆ alcynyle, les quatre derniers groupements pouvant être éventuellement substitués par un ou plusieurs groupements substituants choisis indépendamment parmi :
(a) des atomes d'halogène, -OR⁴ -NR⁵R⁶, -CONR⁵R⁶, -COOR⁷ -NR⁸COR⁹, -SR¹⁰, -SO₂R¹⁰ -SO₂NR⁵R⁶, -NR⁸SO₂R⁹ ;
(b) un cycle à 3-8 chaînons renfermant éventuellement un ou plusieurs atomes choisis parmi O, S, NR⁸ et, lui-même, éventuellement substitué par C₁₋₃ alkyle ou halogène ; ou
(c) un groupement aryle ou un groupement hétéroaryle, dont chacun peut être éventuellement substitué par un ou plusieurs substituants choisis indépendamment parmi des atomes d'halogène ou des groupements cyano, nitro, -OR⁴, -NR⁵R⁶, -CONR⁵R⁶, -NR⁸COR⁹, -SO₂NR⁵R⁶, -NR⁸SO₂R⁹, C₁₋₆ alkyle et trifluorométhyle ;
R⁴ représente hydrogène ou un groupement C₁₋₆ alkyle qui peut être éventuellement substitué par un ou plusieurs groupements substituants choisis indépendamment parmi des atomes d'halogène, -OR¹¹, -NR⁵R⁶, ou un groupement aryle ou un groupement hétéroaryle, l'un ou l'autre pouvant être éventuellement substitué par un ou plusieurs substituants choisis indépendamment parmi des atomes d'halogène ou des groupements cyano, nitro, -OR¹¹, -NR⁵R⁶, -CONR⁵R⁶, -NR⁸COR⁹, -SO₂NR⁵R⁶, -NR⁸SO₂R⁹, C₁₋₆ alkyle ou trifluorométhyle; ou R⁴ représente un atome d'halogène, -OR¹¹, -NR⁵R⁶, ou un groupement aryle ou un groupement hétéroaryle, l'un ou l'autre pouvant être éventuellement substitué par un ou plusieurs substituants choisis indépendamment parmi des atomes d'halogène ou des groupements cyano, nitro, -OR¹¹, -NR⁵R⁶, -CONR⁵R⁶, -NR⁸COR⁹, -SO₂NR⁵R⁶, -NR⁸SO₂R⁹, C₁₋₆ alkyle ou trifluorométhyle ;
R⁵ et R⁶ représentent indépendamment un atome d'hydrogène ou un groupement C₁₋₆ alkyle ou phényle, ou un groupement hétéroaryle, les trois derniers pouvant être éventuellement substitués par un ou plusieurs groupements substituants choisis indépendamment parmi des atomes d'halogène, phényle, -OR¹⁴ et -NR¹⁵R¹⁶, -CONR¹⁵R¹⁶, -NR¹⁵COR¹⁶, SONR¹⁵R¹⁶, -NR¹⁵SO₂R¹⁶,
ou
R⁵ et R⁶, conjointement avec l'atome d'azote auquel ils sont attachés, forment un système de cycle hétérocyclique saturé de 4 à 7 chaînons renfermant éventuellement un autre hétéroatome choisi parmi des atomes d'oxygène et d'azote, lequel système de cycle pouvant être éventuellement substitué par un ou plusieurs groupements substituants choisis indépendamment parmi phényle, -OR¹⁴, -COOR¹⁴, -NR¹⁵R¹⁶, -CONR¹⁵R¹⁶, -NR¹⁵COR¹⁶, -SONR¹⁵R¹⁶, NR¹⁵SO₂R¹⁶ ou un groupement C₁₋₆ alkyle, lui-même éventuellement substitué par un ou plusieurs substituants choisis indépendamment parmi des atomes d'halogène et des groupements -NR¹⁵R¹⁶ et -OR¹⁷ ; R¹⁰ représente un groupement C₁₋₆ alkyle ou phényle, l'un ou l'autre pouvant être éventuellement substitué par un ou plusieurs groupements substituants choisis indépendamment parmi des atomes d'halogène, phényle, -OR¹⁷ et -NR¹⁵R¹⁶ ;
Y est NR²⁰R²¹, OR⁴, SR⁴, un groupement hétéroaryle ou NR⁵R⁶, où R⁵ et R⁶ conjointement avec l'atome d'azote auquel ils sont attachés, forment un système de cycle hétérocyclique saturé de 4 à 7 chaînons renfermant éventuellement un autre hétéroatome choisi parmi des atomes d'oxygène et d'azote, lequel système de cycle pouvant être éventuellement substitué par un ou plusieurs groupements substituants choisis indépendamment parmi phényle, -OR¹⁴, -COOR¹⁴, -NR¹⁵R¹⁶, -CONR¹⁵R¹⁶, -NR¹⁵COR¹⁶, SONR¹⁵R¹⁶, NR¹⁵SO₂R¹⁶ ou un groupement C₁₋₆ alkyle, lui-même éventuellement substitué par un ou plusieurs substituants choisis indépendamment parmi des atomes d'halogène et des groupements -NR¹⁵R¹⁶ et -OR¹⁷; chacun de R⁷, R⁸, R⁹, R¹¹, R¹⁴, R¹⁵, R¹⁶ et R¹⁷ représente indépendamment un atome d'hydrogène ou un groupement C₁₋₆ alkyle ou phényle ;
et R²⁰ et R²¹ sont définis comme R² et R³.

2. Composé selon la revendication 1, **caractérisé en ce que** R¹ représente un groupement benzyle éventuellement substitué.

3. Composé selon la revendication 2, **caractérisé en ce que** R¹ représente benzyle substitué par deux atomes d'halogène.

4. Composé selon l'une quelconque des revendications 1 à 3, **caractérisé en ce que** l'un de R² et R³ est hydrogène et l'autre est C₃₋₄ alkyle substitué par un ou plusieurs groupements hydroxy.

5. Composé selon l'une quelconque des revendications 1 à 4, **caractérisé en ce que** l'un de R² et R³ est hydrogène et l'autre est CH(CH₃)CH₂OH, CH (Et) CH₂OH, C(CH₃)₂CH₂OH ou CH(CH₂OH)₂ .

6. Composé selon l'une quelconque des revendications 1 à 4, **caractérisé en ce que** l'un de R² et R³ est hydrogène et l'autre est CH (CH₃) CH₂OH.

7. Composé selon la revendication 6, sous forme de l'isomère (R).

8. Composé selon l'une quelconque des revendications 1 à 7, **caractérisé en ce que** Y est -NR²⁰R²¹, -OR⁴, -SR⁴, un groupement hétéroaryle ou -NR⁵R⁶, où R⁵ et R⁶, conjointement avec l'atome d'azote auquel ils sont attachés, forment un système de cycle hétérocyclique saturé de 4 à 7 chaînons renfermant éventuellement un autre hétéroatome choisi parmi des atomes d'oxygène et d'azote, lequel système de cycle pouvant être éventuellement substitué par un ou plusieurs groupements substituants choisis indépendamment parmi -OH, -NH₂ ou C₁₋₄ alkyle.

9. Composé selon la revendication 1, choisi parmi :
la 2-[[(2,3-difluorophényl)méthyl]thio]-6-[(2-hydroxyéthyl)aminol-4-[[(1R)-*2*-hydroxy-1-méthyléthyl]amino]-7(8*H*)-ptéridinone;
la 2-[[(2,3-difluorophényl)méthyl]thio]-4-[[(1R)-2-hydroxy-l-méthyléthyl] amino]-6-[(phénylméthyl)amino]-7(8*H*)-ptéridinone;
la 2-[[(2,3-difluoraphényl)méthyl]thio]-4-[[(1R)-2-hydroxy-l-méthyléthyl]amino]-6-7-ptéridinedione ;
la 6-amino-2-[[(2,3-difluorophényl)méthyl]thio]-4-[[(1*R*)-2-hydroxy-1-méthyléthyl]amino]-7(8*H*)-ptéridinone ;
la 2-[[(2,3-difluorophényl)méthyl]thio]-4-[[(1R)-2-hydroxy-1-méthyléthyl]amino]-6-[(1H-imidazol-1-yl)-7(8H)-ptéridinone ;
la 2-[[(2,3-difluorophényl)méthyl]thio]-4-[[(1R)-z-hydroxy-1-méthyléthyl]amino]-6-[(1-méthyl-1H-imidazol-2-yl)thio]-7(8H)-ptéridinone ;
la 2-[[(2,3-difluorophényl)méthyl]thio]-4-[[(1R)-2-hydroxy-1-méthyléthyl]amino]-6-méthoxy-7(8H)-ptéridinone ;
la 2-[[(2,3-difluorophényl)méthyl]thio]-4-[[(1R)-2-hydroxy-1-méthyléthyl]amino]-6-[(3-pyridinylméthyl)amino]-7(8H)-ptéridizinone ;
la 2-[[(2,3-difluorophényl)méthyl]thio]-4-[[(1R)-2-hydroxy-l-méthyléthyl]amino]-6-[[(5-méthyl-2-furanyl)méthyl]amino]-7(8H)-ptéridinone ;
la 2-[[(2,3-difluorophényl)méthyl]thio]-6-[(3R,5S)-3,5-diméthyl-1-pipérazinyl]-4-[[(1R)-2-hydroxy-1-méthyléthyl]amino]-7(8H)-ptéridinone ;
la 2-[[(2,3-difluorophényl)méthyl]thio]-4-[[(1R)-2-hydroxy-1-méthyléthyl]amino]-6-[méthyl[(3-méthyl-5-isoxazolyl)methyl]amino]-7(8H)-ptéridinone ;
la 2-[[(2,3-difluorophényl)méthyl]thio]-4-[[(1R)-2-hydroxy-1-méthyléthyl]amino]-6-[[2-(2-pyrimidinylamino)éthyl]amino]-7(8H)-ptéridinone ;
la 2-[[(2,3-difluorophényl)méthyl]thio]-4-[[(1R)-2-hydroxy-1-méthyléthyl]amino]-6-(4-morpholinyl)-7(8H)-ptéridinone ;
la 2-[[(2,3-difluorophényl)méthyl]thio]-4-[[(1R)-2-hydroxy-1-méthyléthyl]amino]-6-[[2-4-morpholinyl)éthyl]amino]-7(8H)-ptéridinone ;
la 2-[[(2,3-difluorophényl)méthyl]thio]-4-[[(1R)-2-hydroxy-1-méthyléthyl]amino]-6-[(2-méthoxyéthyl]amino]-7(8H)-ptéridinone;
la 2-[[(2,3-difluorophényl)méthyl]thio]-6-[(2-furanylméthyl)amino]-4-[[(1R-2-hydroxy-1-méthyléthyl]amino]-7(8H)-ptéridinone;
la 6-(1-azétidinyl)-2-[[(2,3-difluorophényl)méthyl]thio]-4-[[(1R)-2-hydroxy-1-méthyléthyl]amino] -7(8H)-ptéridinone;
la 2-[[(2,3-difluorophényl)méthyl]thio]-4-[[(1R)-2-hydroxy-1-méthyléthyl]amino] -6-[[(5-méthylpyrazinyl)méthyl]amino]-7(8H)-ptéridinone ;
la 2-[[(2,3-difluorophényl)méthyl]thio]-6-[[2-(2-furanyl)éthyl]amino]-4-[[(1R)-2-hydroxy-1-méthyléthyl]amino]-7(8H)-ptéridinone ;
la 2-[[(2,3-difluorophényl)méthyl]thio]-4-[[(1R)-2-hydroxy-1-méthyléthyl]amino]-6-[[3-(4-morpholinyl)propyl]amino]-7(8H)-ptéridinone ;
la 2-[[(2,3-difluorophênyl)mëthyl]thio]-4-[[(1R)-2-hydroxy-1-méthyléthyl]amino]-6-[[(3-méthyl-5-isoxazolyl)méthyl]amino]-7(8H)-ptéridinone ;
la 2-[[(2,3-difluorophényl)méthyl]thiol-4-[[(1R)-2-hydroxy-1-méthyléthyl]amino]-6-[(3S)-3-hydroxy-1-pyrrolidinyl]-7(8H)-ptéridinone ;
la 2-[[(2,3-difluorophényl)méthyl]thio]-6-[(2-furanylméthyl]thio)-4-[[(1R)-2-hydroxy-1-méthyléthyl]amino]-7(8H)-ptéridinone;
la 2-[[(2,3-difluorophényl)méthyl]thio]-4-[[(1R)-2-hydroxy-1-méthyléthyl]amino]-6-[(2-hydroxypropyl)amino]-7(8H)-ptéridinone;
la 2-[[(2,3-difluorophényl)méthyl]thio]-6-[[2-(diméthylamino)éthyl]thio]-4-[[(1R)-2-hydroxy-1-méthyléthyllaminol-7(8H)-ptéridinone ;
la 2-[[(2,3-difluorophényl)méthyl]thiol-4-[[(1R)-2-hydroxy-1-méthyléthyl]amino]-6-[[(2*S*)-2-hydroxypropyl)amino]-7(8H)-ptéridinone ;
la 2-[[(2,3-difluorophényl)méthyl]thio]-4-[[(1R)-2-hydroxy-1-méthyléthyl]aminol]-6-[(3-hydroxypropyl)amino]-7(8H)-ptéridinone ;
la 2-[[(2,3-difluorophényl)méthyl]thio]-6-[(2-hydroxyéthyl)méthylamino]-4-[[(1R)-2-hydroxy-1-méthyléthyl]amino]-7(8H)-ptéridinone ;
la 2-[[(2,3-difluorophényl)méthyl]thio]-4-[[(1R)-2-hydroxy-1-méthyléthyl]amino]-6-[(5-hydroxy-4-méthyl-4H-1,2,4-triazol-3-yl)thiol-7(8H)-ptéridinone;
la 2- [[(2,3-difluorophényl)méthyl]thio] -6-[(4-(hydroxycyclohexyl)amino]-4-[[(1R)-2-hydroxy-1-méthyléthyl]amino] -7(8H)-ptéridinone ;
la 2-[[(2,3-difluorophényl)méthyl]thio]-4-[[(1R)-2-hydroxy-1-méthyléthyl]amino]-6-(1,3,4-thiadiazol-2-ylthio)-7(8H)-ptéridinone ;
la 2-[[(2,3-difluorophényl)méthyl]thio]-6-[[(1S,4R)-4-hydroxy-2-cyclopentén-2-yl]amino]-4-[[(1R)-2-hydroxy-2-méthyléthyl]amino]-7(8H)-ptéridinone ;
la 2-[[(2,3-difluorophényl)méthyl]thiol-4-[[(1R)-2-hydroxy-l-méthyléthyl]amino] - 6 - [(3R)-3-hydroxy-1-pyrrolidinyl]-7(8H)-ptéridinone;
la 2-[[(2,3-difluorophényl)méthyl]thiol-6-(3-hydroxy-3-méthyl-1-azétidinyl)-4-[[(1R)-2-hydroxy-1-méthyléthyl]amino] -7(8H)-ptéridinone ;
la 6-[(3S)-3-amino-1-pyrrolidinyl]-2-[[(2,3-difluorophényl)méthyl]thio]-4- [[(1R)-2-hydroxy-1-méthyléthyl]amino]-7(8H)-ptéridinone ; et
la 6-[(2-aminoéthyl)thio]-2-[[(2,3-difluorophényl)méthyl]thio]-4-[[(1R)-2-hydroxy-1-méthyléthyl]amino]-7(8H)-ptéridinone.

10. Procédé pour la préparation de :
(a) un composé de formule (I) telle que définie dans la revendication 1, dans laquelle Y est -NR²⁰R²¹, qui comprend le traitement d'un composé de formule (IIA) : 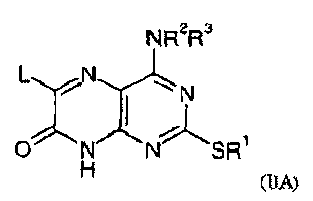 dans laquelle R¹, R² et R³ sont tels que définis dans la formule (I) ou sont des dérivés protégés de celui-ci et L est un groupement partant choisi parmi hydroxy, bromo ou chloro, avec une amine HNR²⁰R²¹, ou
(b) un composé de formule (I) telle que définie dans la revendication 1, dans laquelle Y est OR⁴, qui comprend le traitement d'un composé de formule (IIA), dans laquelle R¹, R² et R³ sont tels que définis dans la formule (I) ou sont des dérivés protégés de celui-ci et L est un groupement partant choisi parmi hydroxy, bromo ou chloro, avec un alcool R⁴OH, ou
(c) un composé de formule (I) telle que définie dans la revendication 1, dans laquelle Y est SR⁴, qui comprend le traitement d'un composé de formule (IIA), dans laquelle R¹, R² et R³ sont tels que définis dans la formule (I) ou sont des dérivés protégés de celui-ci et L est un groupement partant choisi parmi hydroxy, bromo ou chloro, avec un thiol R⁴SH, ou
(d) un composé de formule (I) dans laquelle Y est NR⁵R⁶, qui comprend le traitement d'un composé de formule (IIA), dans laquelle R¹, R² et R³ sont tels que définis dans la formule (I) ou sont des dérivés protégés de celui-ci et L est un groupement partant choisi parmi hydroxy, bromo ou chloro, avec une amine HNR⁵R⁶, ou
(e) un composé de formule (I) dans laquelle Y est un groupement hétéroaryle, qui comprend le traitement d'un composé de formule (IIA), dans laquelle R¹, R² et R³ sont tels que définis dans la formule (I) ou sont des dérivés protégés de celui-ci et L est un groupement partant choisi parmi hydroxy, bromo ou chloro, avec un hétéroarène, ou
(f) un composé de formule (I) telle que définie dans la revendication 1, dans laquelle Y est OH, qui comprend le traitement d'un composé de formule (IIB) : 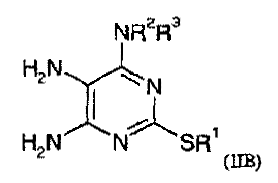 dans laquelle R¹, R² et R³ sont tels que définis dans la formule (I) ou sont des dérivés protégés de celui-ci, avec de l'oxalate de diéthyle, ou
(g) un composé de formule (I) telle que définie dans la revendication 1, dans laquelle Y est NH₂, qui comprend le traitement d'un composé de formule (IIB), dans laquelle R¹, R² et R³ sont tels que définis dans la formule (I) ou sont des dérivés protégés de celui-ci, avec de l'acide iminométhoxyacétique, du chlorhydrate d'ester méthylique, et éventuellement ensuite le procédé (a), (b), (c), (d) et (e), et dans un ordre quelconque:
• l'élimination de tous groupements protecteurs ;
• la formation d'un sel pharmaceutiquement acceptable, d'un solvate ou d'un ester hydrolysable *in vivo.*

11. Composé intermédiaire de formule (IIA), telle que définie dans la revendication 10.

12. Composition pharmaceutique comprenant un composé de formule (I) ou un sel pharmaceutiquement acceptable, un solvate ou un ester hydrolysable *in vivo* de celui-ci, selon l'une quelconque des revendications 1 à 9, en association avec un adjuvant, un diluant ou un support pharmaceutiquement acceptable.

13. Procédé de préparation d'une composition pharmaceutique selon la revendication 12, **caractérisé en ce qu'**il comprend le mélange d'un composé de formule (I), ou d'un sel pharmaceutiquement acceptable, d'un solvate ou d'un ester hydrolysable *in vivo* de celui-ci, selon l'une quelconque des revendications 1 à 9, avec un adjuvant, un diluant ou un support pharmaceutiquement acceptable.

14. Composé de formule (I), ou sel pharmaceutiquement acceptable, solvate ou ester hydrolysable *in vivo* de celui-ci, selon l'une quelconque des revendications 1 à 9, destiné à être utilisé en thérapie.

15. Utilisation d'un composé de formule (I), ou d'un sel pharmaceutiquement acceptable, d'un solvate ou d'un ester hydrolysable *in vivo* de celui-ci, selon l'une quelconque des revendications 1 à 9, dans la fabrication d'un médicament destiné à être utilisé en thérapie.
